# EUROPEAN PATENT SPECIFICATION

(11) **EP 4 200 875 B1**
(45) Date of publication and mention of the grant of the patent: **07.10.2026**
(21) Application number: 21765919.2
(22) Date of filing: 20.08.2021
(51) Int. Cl.: G16H 50/30, G01N 33/68, G16H 50/20

(54) **COMPUTING DEVICE FOR ESTIMATING THE PROBABILITY OF MYOCARDIAL INFARCTION**
RECHENVORRICHTUNG ZUR SCHÄTZUNG DER WAHRSCHEINLICHKEIT EINES HERZINFARKTS
DISPOSITIF INFORMATIQUE POUR ESTIMER LA PROBABILITÉ D'INFARCTUS DU MYOCARDE

(30) Priority: 24.08.2020 EP 20192459
(43) Date of publication of application: 28.06.2023
(73) Proprietor: ART-EMIS Hamburg GmbH, 21224 Rosengarten (DE)
(72) Inventor: BLANKENBERG, Stefan, 20251 Hamburg (DE); ZELLER, Tanja, 22529 Hamburg (DE); OJEDA ECHEVARRIA, Francisco, Miguel, 22145 Hamburg (DE); NEUMANN, Johannes, 20255 Hamburg (DE); ZIEGLER, Andreas, 7265 Davos Wolfgang (CH); TWERENBOLD, Raphael, 20457 Hamburg (DE)
(74) Representative: Eisenführ Speiser
(86) International application number: PCT/EP2021/073193
(87) International publication number: WO 2022/043229

(56) References cited:
- WO-A1-2017/173353
- JOHANNES T. NEUMANN ET AL: "Application of High-Sensitivity Troponin in Suspected Myocardial Infarction", NEW ENGLAND JOURNAL OF MEDICINE, vol. 380, no. 26, 27 June 2019 (2019-06-27), pages 2529 - 2540, XP055764818, ISSN: 0028-4793, DOI: 10.1056/NEJMoa1803377
- FRED S APPLE ET AL: "Cardiac troponin assays: guide to understanding analytical characteristics and their impact on clinical care", CLINICAL CHEMISTRY (BALTIMORE, MD.), 31 December 2016 (2016-12-31), England, pages 73 - 81, XP055764504, Retrieved from the Internet <URL:http://hytestchina.com/uploadfiles/2017/09/20170905140512512.pdf> [retrieved on 20210113], DOI: 10.1373/clinchem.2016.255109

## Description

### TECHNICAL FIELD

The present disclosure generally relates to a computing device for estimating the probability of myocardial infarction.

### BACKGROUND

A myocardial infarction typically occurs when blood flow to a part of the heart stops or decreases, which causes damage to the heart muscle. One of the most common symptoms of myocardial infarction is chest pain or discomforts in the shoulder, arm, back, neck or jaw. Other symptoms may include shortness of breath, nausea, feeling faint, a cold sweat or feeling tired.

The assessment of patients potentially suffering from acute myocardial infarction is one of the most difficult challenges in medical practice. Patients who have been incorrectly ruled-out and sent home, have a higher mortality rate than correctly hospitalized patients. In contrast, due to the lack of hospital resources and the high costs involved in hospitalization, hospitals have to carefully diagnose, whether a patient showing symptoms has a potential risk of myocardial infarction.

Therefore, a solution is needed so that medical facilities are able to estimate, with a high degree of certainty, whether the patient has a myocardial infarction or not, to provide efficient and correct treatment for those patients in need.

A fast and reliable detection or exclusion of myocardial infarction is of high clinical relevance. In addition to clinical evaluation and the 12 Channel ECG, cardiac troponin plays a central role in the diagnosis of myocardial infarction. However, troponin assays previously used for measuring a cardiac troponin had low first-draw sensitivities due to the time required for a detectable rise in measurable troponin concentrations, which lead to a so-called troponin blind interval. To overcome this limitation, high-sensitivity troponin assays have been developed that shortened the troponin blind interval significantly in the first hours after the first appearance of symptoms of myocardial infarction. This improvement led to the development of rapid triage-algorithms, which recommend a serial measurement troponin of cardiac troponin markers every 1, 2 and 3 hours.

High-sensitivity troponin assays as specified herein may refer to assays for measuring cardiac troponin. Cardiac troponin (cT) is a protein complex that is released from the muscle cells of the heart into the blood in the event of damage during a heart attack. Cardiac troponin may comprise three measureable troponin markers including C (cTne), T (cTnT) and I (cTnI) that control the calcium mediated interactions between actin and myosin in cardiac and skeletal muscles. The Troponin markers cTnI and cTnT are specific to cardiac muscles, unlike troponin cTnC which may be associated with both cardiac and skeletal muscles. Therefore, for estimating the presence of myocardial infarction measuring the troponin markers cTnT and cTnI may be of high relevancy.

WO 2017/173353 A1 discloses decision tree based systems and methods for estimating the risk of acute coronary syndrome (ACS) in subjects suspect of having ACS. In particular, systems and methods are provided that employ additive decision tree based algorithms to process a subject's initial cardiac troponin I or T (cTnI or cTnT) concentration, a subject's cTnI or cTnT rate of change, and at least one of the following: the subject's age, the subject's gender, the subject's ECG value, the subject's hematology parameter value, to generate an estimate risk of ACS.

However, what is needed is an improved approach for detection or exclusion of myocardial infarction for a subject.

### SUMMARY

The above identified objectives are solved by a computing device for estimating the probability of myocardial infarction and a corresponding system as defined in the independent claims. Preferred embodiments are defined in the dependent claims.

The disclosure refers to a computing device for estimating the probability of myocardial infarction. The computing device comprises a receiver configured to receive a set of vital parameters of a subject, the set of vital parameters including troponin data of the subject, the troponin data of the subject being measured using an initial troponin assay, a selector configured to select a troponin assay from a set of troponin assays, a repository configured to provide a plurality of estimation modules corresponding to the selected troponin assay, wherein the plurality of estimation modules constitute a super learner module, and at least one processor configured to use the plurality of estimation modules to estimate the probability of myocardial infarction of the subject using the set of vital parameters including the troponin data of the subject.

The computing device enables an estimation of probabilities of myocardial infarction using vital parameters and troponin data measured using a variety of troponin assays. For each of the set of troponin assays, the plurality of estimation modules enable a fast and reliable estimation for the measured troponin data. The computing device is configured via the selector that relates a selected troponin assay to the plurality of estimation modules corresponding to the selected troponin assay. Hence, the computing device enables a fast and reliable estimation of myocardial infarction for a subject due to pre-defined estimation modules and further provides for improved flexibility with regard various troponin assays that are used to measure troponin data. The selection of troponin assays further allows for an update of the computing device to cover new developments of troponin assays. Furthermore, the repository may include estimation modules that take into account future improvements in estimating acute myocardial infarctions.

The estimation of probabilities of myocardial infraction may rely on data retrieved from a database with a plurality of datasets, wherein each dataset may be associated with a subject. Each dataset may include troponin features and/or non-troponin features. The non-troponin features and corresponding values of the non-troponin features may include age (in years), sex (male/female/diverse), heart failure (yes/no), history of coronary artery disease (yes/no), family history of coronary artery diseases (yes/no), atrial fibrillation (yes/no), systolic blood pressure (mm Hg), hypertension (yes/no), hyperlipoproteinemia (yes/no), diabetes (yes/no), smoking status (Smoker/Non-Smoker), estimated glomerular filtration rate (eGFR) (ml/min), ECG ischemic signs (yes/no), and/or symptom of myocardial infarction onset time greater than or equal to 3h (yes/no). The troponin features may include a measured cardiac troponin marker. A measured cardiac troponin may be a measurement of the troponin marker cTnI or the troponin marker cTnT. The troponin features may further include a troponin assay used for measuring the troponin marker. The troponin assay may be a high-sensitivity troponin assay. The high-sensitivity troponin assay may be one of a set of high-sensitivity troponin assays. Each one of the set of high-sensitivity troponin assays may be provided from a different manufacturer. At least one of the high-sensitivity troponin assays may correspond to a high-sensitivity troponin assay for measuring the troponin marker cTnI. At least one of the set of high-sensitivity troponin assays may correspond to high-sensitivity troponin assays for measuring the troponin marker cTnT. Each dataset may further include a myocardial feature. A myocardial feature may indicate whether a subject associated with a dataset may have a myocardial infarction or not for the non-troponin and troponin features included in the dataset. For the myocardial features a first value, such as 1, may indicate that the respective subject has a myocardial infarction, wherein a second value, such as o, may indicate that the subject does not have a myocardial infarction.

The troponin features may further include an initial troponin measurement. An initial troponin measurement may include a measurement value of the troponin marker, a timestamp of the measurement and information indicating whether the measurement value at a time of the timestamp was greater than or equal to a limit of detection (LOD). A LOD may describe an extreme value corresponding to a high-sensitivity troponin assay up to which the measurement may be reliably detected. The troponin features may further include a second troponin measurement. A second troponin measurement may include a second troponin measurement value of the troponin marker, a timestamp of the second measurement and information indication whether the second troponin measurement value at the timestamp of the second troponin measurement was greater than or equal to the LOD. If troponin features include an initial and a second troponin measurement, troponin features may further include a troponin rate that indicates a change of the troponin measurement value between the initial and the second troponin measurement value. If a troponin measurement includes an initial and a second troponin measurement, the troponin measurement may further include a time difference between the timestamp of the initial and the timestamp of the second troponin measurement.

Troponin measurement values may be represented in nanograms per litre of blood units [ng/L]. Each troponin measurement value included in the troponin features may have been log-transformed using the natural logarithm to reduce skewness. The log-transformed troponin measurement values and LODs of the troponin features may have been multiplied by 10 to obtain better scaling of estimates.

The database may be the Biomarker in Acute Cardiac Care (BACC) database. However, it is to be understood that any other suitable database can be used, such as the stenoCardia database. The BACC may comprise datasets associated with at least 2307 subjects. The stenoCardia database may comprise datasets associated with at least 1818 subjects. The database may include subjects with ST-segment elevation myocardial infarction (STEMI). When excluding subjects with STEMI, the BACC database may comprise datasets associated with at least 2187 subjects. When excluding subjects with STEMI, the stenoCardia database may comprise datasets associated with at least 1688 subjects. To assign missing values in either the troponin and/or non-troponin features of a dataset, multiple imputation may be performed by using values from multivariate imputation by chain equations (mice) to assign the missing values. When performing multiple imputation, a plurality of datasets that may include non-troponin and troponin features based on mice may be generated randomly. One of the generated datasets may be selected to assign the missing values.

Hence, when estimating the probability of myocardial infarction, the computing device is not limited to analyzing one or a limited set of parameters only, but can rather include various relevant factors from the database, to provide a precise estimation of an acute myocardial infarction.

The receiver of the computing device according to the present disclosure may receive the set of vital parameters in response to a request for retrieving the set of vital parameters of the subject. The request may be sent from the computing device. The request may be a web-protocol request or a database query. The web-protocol request may be an HTTP request, a WebRTC request, a QUIC request, or any other request based on a standard or dedicated communication protocol. The set of vital parameters may be received by the receiver in a data interchange format. According to one example, the data interchange format may be a JSON format, XML, or any other suitable data interchange format.

The received set of vital parameters may comprise non-troponin features, such as non-troponin features of an underlying database. The initial troponin assay may correspond to a high-sensitivity troponin assay. The initial troponin assay may refer to a troponin assay provided by a manufacturer.

The selector may select the troponin assay in response to a manual, pre-set or an automated selection of the troponin assay. At least one troponin assay of the set of troponin assays may be a high-sensitivity troponin assay, such as a high-sensitivity assay for measuring the troponin marker cTnT or a high-sensitivity assay for measuring the troponin marker cTnI. At least some of the set of troponin assays may be provided from a different manufacturer. Each troponin assay may specify one or more of a name of the troponin assay, the manufacturer of the troponin assay, a troponin marker the troponin assay may measure and/or an LOD of the troponin assay.

The selection of the troponin assay may be a manual user interaction with the computing device. The selector may provide the set of troponin assays via a user interface to the user, thereby enabling the user to select a troponin assay from the set of troponin assays via the user interface.

The selection of the troponin assay may also be controlled by a central instance of a medical facility. The central instance may pre-set a troponin assay that the medical facility uses and the selector may automatically select the pre-set troponin assay. The selection of the troponin assay may also be automated based on an analysis of the troponin data of the set of vital parameters.

It is to be understood that if the troponin assay is selected manually by a user or pre-set by a central instance or automated, the selection must correspond to the initial troponin assay to enable the computing device to estimate a correct probability. Thus, the selected troponin assay corresponds to the initial troponin assay. Yet, the degree of freedom with regard to a broad variety of the troponin assays enables a flexible and fast estimation of probabilities even for different troponin assays since the computing device is pre-configured with several set of estimation modules for the respective troponin assays.

The repository may be a storage of the computing device to store the plurality of estimation modules. Each stored plurality of estimation modules may be fitted with datasets of the database associated with a troponin assay from the set of troponin assay. Hence, each stored plurality of estimation modules may be associated with a troponin assay from the set of troponin assays. When the selector selects a troponin assay from the set of troponin assays, the plurality of estimation modules associated with the selected troponin is provided by the repository.

The super learner module is defined by a combination of the plurality of estimation modules. Each estimation module may be a machine of the super learner module. The machines of the super learner module may be initially determined based on an estimation performance of the individual machines with regard to data of a suitable database, such as the previously described BACC database. A set of best performing or ranked machines is selected for the super learner module. Accordingly, the plurality of estimation modules constituting the super learner module corresponds to the selected best performing or ranked machines. As an example, the plurality of estimation modules can be combined by an optimal convex combination of weights of the plurality of estimation modules. The at least one processor may input the set of vital parameters into the plurality of estimation modules, such that the set of vital parameters is provided to and input into each estimation module of the plurality of estimation modules of the super learner module. In response, each estimation module may estimate the probability of myocardial infarction for the set of vital parameters. The probability of myocardial infarction of the subject may be an average value of the estimated probabilities of each estimation module of the plurality of estimation modules. As an alternative, a highest probability estimated by an estimation module of the plurality of estimation modules may be the probability of myocardial infarction of the subject. Other combinations are encompassed by the present disclosure.

The at least one processor may provide the probability of myocardial infarction via a user interface of the computing device to a user. The at least one processor may analyze the probability and perform validity or plausibility checks and other reliability processing before providing the probability via the user interface.

The computing device may be used by a medical professional onsite to directly analyze vital parameters of the subject. Respective computations may be performed on the computing device. However, at least some or all of the functionality may be provided as a cloud service or a web service, for example, as a web application or a software as a service application. The computing device may connect to the service provider to perform the respective functionality and receive results. The cloud service or the web service may be provided at a central instance of a medical facility, such as one or more servers providing the service hosted by the medical facility, which may act as a local service provider, or as a remote service provider. Any sensitive data transmitted to or received from the service provider may be encrypted. For example, when using the computing device with a service provider, the receiver may receive the set of vital parameters from the service provider. Moreover, the selection of the troponin assay may be performed via the service provider and submitted to the selector of the computing device. In this case, the repository may only provide identifications of the plurality of estimation modules, which are shared with the service provider or otherwise verified, and all computations related to estimating the probability of myocardial infarction may be performed by the service provider. The at least one processor may estimate and provide the probability based on results received from the service provider. Hence, the at least one processor may be configured to use the plurality of estimation modules in that identifications of the estimation modules and/or of the super learner module are submitted to the service provider, which then estimates the probability of myocardial infarction of the subject using the set of vital parameters including the troponin data of the subject. The results may be transmitted back from the service provider to the computing device.

In yet another embodiment, the computing device may be the computing device hosted by the service provider to provide a respective cloud service to connected terminal devices, which may be operated by medical professionals at a medical facility, for example. Hence, the computing device may provide a cloud service or a web service, for example, as a web application or a software as a service application to connected terminal devices.

In one embodiment, the troponin data includes at least one troponin measurement of a troponin marker, the troponin measurement including a measurement value of the troponin marker and a timestamp of the troponin measurement. The at least one troponin measurement may correspond to an initial troponin measurement and/or a second troponin measurement according to troponin features of the database. The measurement value may correspond to an initial troponin measurement value or a second troponin measurement value according to troponin features of the database. The troponin data may further include a troponin rate that indicates a change of the troponin measurement value between the initial troponin measurement and the second troponin measurement.

In another embodiment, the at least one processor is further configured to estimate a prediction interval for the probability of myocardial infarction. Given observed probabilities with variabilities, the prediction interval is an estimate of an interval in which a future observation will fall, with a certain probability, given the observed probabilities. Prior to or after the probability of myocardial infarction was estimated, the user may interact with the user interface to specify whether the prediction interval should be estimated. If the user confirms the request, the processor may estimate the prediction interval for the estimated probability. Additionally or as an alternative, the processor may automatically estimate the prediction interval for the estimated probability.

In one embodiment, the prediction interval is estimated by setting a coverage level, drawing equal-sized folds of datasets in at least a part of a database for cross-validation, forming cross-validated observations with prediction variability for each equal-sized fold, determining a mean and standard deviation of the cross-validated observations, and estimating the prediction interval using the coverage level, and the mean and standard deviation of the created observations.

The coverage level may represent a percentage indicating how likely it may be that the probability of myocardial infarction may lie within the prediction interval. The estimated prediction interval may be based on the coverage level. In some embodiments, the coverage level may be set to at least 80, preferably 85, 90, 95 or 99, wherein 95 may be preferred. However, it should be understood that the coverage level may also be set to any number between 0 and 100.

Each of the equal-sized folds may comprise the same amount of datasets. The datasets of the equal-sized folds may be drawn randomly without reputation from the database for cross-validation. The database for cross-validation may be or at least partly correspond to the database. In one example, the number of drawn equal-sized folds may be at least 5, preferably 5 to 30 equal-sized folds, most preferably 5, 10, or 20 equal-sized folds. A higher number of equal-sized folds may lead to less variability between the randomly drawn datasets of the equal-sized folds, while a smaller number of equal-sized folds may lead to a higher variability between the datasets of the equal-sized folds. However, a smaller number of equal-sized folds may result in a faster computation time for estimating the prediction interval, while a higher number of equal-folds may result in longer computation time for estimation the prediction interval.

For each equal-sized fold, cross-validated observations with prediction variability may be formed using fold cross-validation. In fold cross-validation, a respective equal-sized fold of the equal-sized folds is left-out when training an estimation module. Accordingly, an estimation module may be trained using the equal-sized folds without the respective equal-sized fold. When the estimation module is trained, the respective equal-sized fold may be used to make predictions for data in the equal-sized fold. This process may be repeated for each equal-sized fold, wherein in each iteration a different respective equal-sized fold is used so that cross-validated observations may be formed for each equal-sized fold. A cross-validated observation may represent an estimation of myocardial infarction with prediction variability.

For each equal-sized fold, cross-validated observations may be retrieved and collected until cross-validated observations are formed for each equal-sized fold. A mean and standard deviation of the cross-validated observations may be determined when cross-validated observations are formed for each equal-sized fold.

The prediction interval may be estimated using the coverage level, and the mean and standard deviation of the created observations. In some embodiments, estimating the prediction interval may include estimating a quantile of a distribution based on the coverage level. The distribution may be a standard distribution, a Poisson distribution, a standard normal distribution or a Student's t-distribution. An upper bound of the prediction interval may be defined as the mean average value plus the estimated quantile multiplied with the standard deviation. A lower bound of the prediction interval may be defined as the mean average value minus the estimated quantile multiplied with standard deviation.

The prediction interval may be estimated by defining quantiles of the formed cross-validated observations. One of the defined quantiles may be based on the coverage level, while another quantile may be based on counter-probability of the coverage level. The counter-probability may be 1 minus the coverage level. The coverage level and the counter-probability may be divided by a factor. The factor may be between 1.1 and 4, most preferably between 1.5 and 2.5, wherein 2 may be most preferred. The factor may be used for optimizing ranges of the prediction interval. The quantile related to the coverage level may be the upper bound of the prediction interval. The quantile based on the counter-probability may be the lower bound of the prediction interval.

Since the prediction interval is dependent on the coverage level, a coverage level may result in a broad prediction interval, which may be too wide. For a too wide prediction interval nearly all future estimated probabilities of myocardial infarctions may fall within the range of the prediction interval. However, if the coverage level is set to lower value, the range of the prediction interval between the upper and lower bound may be too narrow, meaning that barely any in future estimated probabilities of myocardial infarctions will fall within the prediction interval. Values outside the upper and lower bounds of the prediction interval may represent variances for the estimated probability. The estimated prediction interval and/or ranges outside the upper and lower bounds of the prediction interval may be provided via the user interface of the computing device to the user.

In a preferred embodiment, forming cross-validated observation comprises, for each equal-sized fold, training each of the plurality of estimation modules using the equal-sized folds without the respective equal-sized fold, making predictions for all data in the respective equal-sized fold, estimating residuals for all data in the respective equal-sized fold using the predictions, and randomly drawing a number of data without replacement from the respective equal-sized fold, to form the cross-validated observations plus prediction error using the fold-specific predicted observation and the respective residual of the randomly drawn data.

Forming cross-validated observations may be repeated for the respective equal-sized fold for each of the plurality of estimation modules. When forming cross-validated observations for each equal-sized fold, the respective estimation module may be trained using the equal-sized folds without the respective equal-sized fold. After the respective estimation module was trained, the respective equal-sized fold may be used to make predictions for all data in the respective equal-sized fold. When making predictions for all data in the respective equal-sized fold, the respective equal-sized fold is inputted into the respective estimation module.

For each of the predictions, residuals may be estimated. A residual may be estimated by subtracting a value of a myocardial feature of a dataset from the respective equal-sized fold with the estimated probability of myocardial infarction associated with the dataset. Each estimated residual may be stored in a temporal data structure, such as in an array, a list, a dictionary, or a table, wherein the temporal data structure may be associated with the respective estimation module and the respective equal-sized fold.

The set of vital parameters may represent a new data point. The cross-validated observations may be formed for each respective equal-sized fold for each of the plurality of estimation modules. For a respective estimation module, a random number of data may be drawn randomly without reputation from the respective equal-sized fold. In some embodiments, the number of randomly drawn data may be less than or equal to a relation between the number of datasets included in the database for cross-validation and the number of drawn equal-sized folds. Preferably, at least one, at least a half, at least a third, at least a quarter, at least a tenth, or all of the data of the respective equal-sized fold may be drawn randomly. However, it is to be understood that any number of data may be drawn randomly from the respective equal sized fold. For each of the randomly drawn data, the respective residual of the randomly drawn data may be used to from a cross-validated observation by adding the respective residual together with the estimation of myocardial infarction of the new data point. The respective residual may be retrieved from the temporal data structure, such as a pre-computed table, associated with the respective estimation module and the respective equal-sized fold.

In a preferred embodiment, residuals are stored in one or more pre-computed tables in the repository. At least one of the one or more pre-computed tables may be retrieved from a server and stored in the repository. The pre-computed tables may be associated with an equal-sized fold and/or an estimation module. Thus, instead of estimating residuals for all data in the respective equal-sized fold using the predictions, the residuals may be retrieved from the pre-computed tables.

In another embodiment, the super learner module corresponds to a 1 measurement model if the troponin data includes one troponin measurement, and the super learner module corresponds to a 2 measurement model or to a 1 measurement model if the troponin data includes at least two troponin measurements. In order to determine whether a 1 measurement model or a 2 measurement model is to be provided by the repository, the at least one processor may scan the received troponin data included in the received set of vital parameters of the subject. If the troponin data includes one troponin measurement, a request to provide the 1 measurement model may be sent to the repository. If the troponin data includes at least two troponin measurements, a request to provide either the 1 measurement model or preferably the 2 measurement model may be sent to the repository.

In a preferred embodiment, the 1 measurement model comprises estimation modules of differing types including one or more of a gradient boosting machine, a generalized additive machine and a generalized boosting machine. The machines of the 1MM may be machines selected from a set of machines, in any combination. The set of machines may comprise a logistic regression machine, a logistic regression machine with backward elimination, a logistic regression machine with univariate screening, a component-wise gradient boosting machine, a generalized additive model machine, an elastic net logistic regression machine, a multivariate adaptive regression splines machine, a gradient boosting machine, a random forest machine, a random forest machine with feature selection and/or a support vector machine. The 2 measurement model may comprise estimation modules of differing types including one or more of a generalized additive machine, a generalized boosting regression machine and a random forest machine. The machines of the 2MM may be machines selected from the set of machines. However, it is to be understood that the machines of the 2MM may include or other further machines from the set of machines, in any combination.

In a preferred embodiment, machines for the super learner module are estimated by computing performance estimates using full features, performing feature selection, computing performance estimates using reduced features, comparing the computed performance estimates, and selecting one or more machines for the super learner module.

The machines for the super learner module may be estimated for the 1MM and/or for the 2MM. The full features may comprise datasets of the database, including the non-troponin features, the troponin features and myocardial features of the database. When estimating machines for the 1MM, the troponin features of the full features may only include the initial troponin measurements of the troponin features of the database.

When estimating machines for the 2MM, the troponin features of the full features may include initial troponin measurements and second troponin measurements of the troponin features of the database. The full features may be divided into full feature sets. A full feature set may include datasets that are associated with one troponin assay. Each of the full feature sets may be divided into a number of partitions. In one example, the number of partitions may be at least 5 partitions, preferably 5 to 50 partitions, most preferably, 10, 15, 20, 25, or 30 partitions, such as most preferably 10 partitions. However, it is to be understood that any number of partitions may be used to suitably estimate the machines for the super learner module. The partitions may be drawn randomly without reputation from the full feature set.

Performance estimates may be computed for each troponin assay included in the database and for each machine from the set of machines. The performances estimates may be computed for a full feature set for one machine. When computing a performance estimate for one machine for a troponin assay, a reduced number of partitions of a full feature set may be used to fit the machine. As an example, this may include 9 of 10 partitions. However, it is to be understood that any reduced number of partitions or a different fraction of the reduced number of partitions may be used, such as 6, 7, or 8 of 10 partitions. The reduced number may be scaled to the appropriate total number of partitions in an appropriate manner. The remaining one or more partitions not used to fit the machine may be used to compute a performance estimate. This may include a single remaining partition. The performance estimate may be based on an average score and an average error-rate based on the average score. Scores may include an Area Under the Curve (AUC) score and/or a Brier score based on the AUC score. However, it is to be understood that these scores are examples only and any other suitable score can be used for performance estimate. The AUC score and the Brier score may be obtained a number of times for one machine. In one particular example, the number of times may be at least 5 times, preferably 5 to 25 times, most preferably, 10, 15, 20, or 25 times, such as most preferably 10 times. In a particularly preferred example, the performance estimate for a full feature set for one machine may be an average AUC score and an average Brier score based on the 10 obtained AUC scores and Brier scores. However, it is to be understood that the scores may be obtained in any number for performance estimate.

Performing feature selection may include selecting features and values corresponding to age and gender features of the non-troponin features of the full features, selecting one or more features and corresponding values that may be included at least a number of times in at least one partition of one full feature set, such as 5 times. However, it is to be understood that any other suitable number may be chosen. In one example, eGFR features and values may be discarded from the selected features. If a troponin measurement value is selected as a feature, a LOD associated with the troponin measurement may be selected as a feature. If a LOD is selected as a feature, a corresponding troponin measurement value may be selected as a feature.

The reduced features may correspond to the selected features. A reduced feature set may be based on a full feature set. One or more retrieved datasets of a full feature set by using feature selection may be the reduced feature set that is based on the full feature set. Each of the reduced feature sets may be divided into a number of partitions, such as 10 partitions or any other suitable number of partitions, as discussed above. Performance estimates for the reduced features are computed in the same way as the performance estimates for the full features. The logistic regression machine with backward elimination, logistic regression machine with univariate screening and the random forest machine with feature selection from the set of machines may not be used for computing the performance estimates using reduced features.

The performance estimates for the full features may be compared with the performance estimates for the reduced features. Selecting machines for the super learner module may be based on the computed AUC scores and/or Brier scores for the full feature sets and/or the reduced feature sets. In some embodiments, if a highest AUC score and/or the lowest Brier score was achieved either for the full feature sets or for the reduced feature sets, the computed AUC scores and/or Brier scores of a respective feature set may be further reviewed. In some embodiments, AUC scores and/or Brier scores computed for the reduced feature sets may be preferred for further review regardless of the computed AUC scores and/or Brier score for the full feature sets. In other embodiments, AUC scores and/or Brier scores computed for the full feature sets may be preferred for a further review regardless of the computed AUC scores and/or Brier score for the reduced feature sets. Comparing the performance estimates may include ranking the computed performance estimates for each machine from the set of machines used for computing the performance estimates for the full features and reduced features. Ranking may be performed for each machine from the set of machines and for each troponin assay included in the database. Ranking may be based on the computed AUC scores and Brier scores for each computed performance estimate for each machine. In particular, the AUC scores with a highest value for a machine may have a highest rank. The Brier score with a lowest value for a machine may have a highest rank. The ranked AUC scores and Brier scores for a machine for each assay may each be accumulated.

The highest ranked machine may be the machine that achieved the lowest value when accumulating the ranks for both the AUC scores and the Brier scores. The highest ranked machine may be selected as a machine for the super learner module. When selecting the highest ranked machine, ranking based on the AUC scores may be preferred.

Comparing the computed performance estimate may further include analyzing data resulting from scatterplots and/or correlations. A scatterplot and/or a correlation may be analyzed between two machines for each assay included in the database. One of the two machines may be the highest ranked machine. Comparing may be performed between the highest ranked machine and a subsequent following ranked machine. A subsequent following machine may be a machine that follows the highest ranked machine based on the AUC score rankings and/or the Brier score rankings. When computing a correlation and a scatterplot for one troponin assay for one machine, a remaining partition of a reduced feature set associated with the troponin assay may be inputted into each of the highest ranked machine and the subsequent following machine. The scatterplots and/or correlations may be based on probabilities that the highest ranked machine and the subsequent following machine estimated for the remaining dataset. When analyzing scatterplots and/or correlations for the reduced features, the two machines may have been fitted during the computing of performance estimates for the reduced features. If a correlation between the two machines is substantially 1, the subsequent following machine may not be considered as a machine for the super learner module. If a scatterplot between two machines forms an ideal graph, the subsequent following machine may also not be considered as a machine for the super learner module. If a subsequent following machine may not be considered as a machine for the super learner module, analyzing scatterplots and/or correlations may be repeated with a machine subsequently following the subsequent following machine until a pre-estimated number of machines is reached. For example, the number may be an uneven number. Having an uneven number of machines for the super learner module may enable a majority decision of the combined machines in the super learner module.

In a preferred embodiment, a set of equal weights may be estimated for the super learner module, preferably by combining the machines selected as machines for the super learner module and performing a grid-search. The grid-search may comprise a search for optimal parameters. The search may be an exhaustive search, including a plurality of searches for an optimal parameter between a range from a starting point until an endpoint in predefined steps. In one example, the range may be from 0 to 1. Another starting point may be 0.1, 0.2 or 0.3. Another endpoint may be 0.9, 0.8 or 0.7. A predefined step may preferably be 0.005. Another predefined step may also be 0.001 or 0.01. However, it is to be understood that other starting points and/or endpoints between 0 and 1 and other step ranges may be used as well.

The database may be divided into 10 partitions. In one example, the number of partitions may be at least 5 partitions, preferably 5 to 50 partitions, most preferably, 10, 15, 20, 25, or 30 partitions. However, it is to be understood that any number of partitions may be used. The partitions may all comprise the same amount of datasets and may be drawn randomly. In each search from the plurality of searches the machines selected for the super learner module may be fitted with at least some of the partitions. For example, if a database was divided into 5 partitions, 4, 3, 2 or 1 partition of the 5 partitions may be used for fitting the machine. If a database is divided into 50 partitions, any number of 49 to 1 partitions of the 50 partitions may be used for fitting the machine. However, it is to be understood that the same or a similar concept may apply if the database is divided into 10, 15, 20, 25, or 30 partitions.

One or more remaining partitions not used to fit the super learner module may be inputted into the machines to estimate the AUC score. In one example, the number of estimated AUC scores may be at least 1, 2, 3, 4, or 5 AUC scores. However, most preferably, 10 AUC scores may be estimated in each search. However, it is to be understood that any number of AUC scores may be estimated in each search. Based on the estimated AUC scores, an average AUC score may be computed. An AUC score of the super learner module may be an average value of computed AUC scores of each machine the super learner module comprises. The optimal parameters may be an optimal convex combination for which a computed average score is the highest, such as the AUC score.

In one embodiment, the super learner module may be fitted with the set of equal weights. Preferably, a performance between the super learner module fitted with equal weights, the super learner module not fitted with equal weights, and each machine of the set of machines used for computing the performance estimates may be compared.

In a preferred embodiment, one or more super learner modules are fitted by a server, using the set of equal weights.

In some embodiments, if AUC scores and/or Brier scores computed for the reduced feature sets are used, the reduced feature sets and the machines used for computing the performance estimates of the reduced features may be used to compute scores for comparing. A module of the super learner module fitted with equal weights and the super learner module that estimates a higher AUC score and/or a lower Brier score may be considered as the best performing machine. The performance between the super learner module fitted with equal weights and the super learner module may be evaluated using an external database not used for configuring and selecting the machines for the super learner module.

In yet another embodiment, the computing device further comprising an extension component configured to add a further troponin assay to the set of troponin assays, wherein a plurality of further estimation modules corresponding to the further troponin assay is stored in the repository. Each of the estimation modules of the plurality of further estimation modules may be trained with data associated with the further troponin assay. Preferably, each of the machines of the 1MM and each of the machines of the 2MM may be trained with data associated with the added troponin assay. The trained plurality of further estimation modules may be retrieved from a remote storage or may be transmitted or otherwise provided to the computing device. The computing device may also retrieve the data associated with the further troponin assay and at least partially train the estimation modules of the plurality of further estimation modules. The data associated with the further troponin assay may be retrieved from the database or another database including respective data.

The extension component may be triggered manually via the user interface. Additionally or as an alternative, the extension component may be triggered remotely by a central instance of a medical facility, or automatically upon detection that a new troponin assay is used. The extension component may request information on the further troponin assay, for example, via the user interface. The information may include one or more of a name of the further troponin assay, a name of the manufacturer of the further troponin assay, a troponin marker the further troponin assay may measure, and/or an LOD of the further troponin assay, in any combination.

In another embodiment, the computing device further comprising a preselector component configured to preselect a troponin assay corresponding to the initial troponin assay based on at least the troponin data. The preselector component may be triggered when a set of vital parameters is received by the receiver. The preselector component may scan the troponin data of the set of vital parameters. The scanning may include estimating at least one troponin assay from the set of troponin assays that was most likely used for measuring the troponin measurements included in the troponin data of the set of vital parameters. A most likely troponin assay may be a troponin assay with a matching manufacturer. Additionally or as an alternative, the most likely troponin assay may be able to measure the same troponin marker as for the troponin data. After estimating the most likely troponin assay, the selector may select or preselect the most likely used troponin assay. The preselector component may further estimate a matching score to indicate correctness of preselection. The preselection and/or the matching score may be provided via the user interface to the user for confirmation. Subsequently, the selector may either directly or responsive to user input select trigger the repository to provide the plurality of estimation modules associated with the preselected troponin assay. Thus, the preselector component enables an automatic selection of a troponin assay based on the received set of vital parameters.

According to a preferred embodiment, the computing device further comprises a user interface, wherein the user interface is configured to receive input of a user and provide at least the estimated probability to the user. The user interface may handle user interactions with the computing device in any suitable manner, such as via touching, clicking, voice, typing and/or gesture events. The user interface may be a graphical user interface (GUI) or any other user interface of a suitable modality. Responsive to user interaction a troponin assay may be selected.

**In** one embodiment, the user interface is further configured to receive information for adding a further troponin assay to the set of troponin assays. This may be triggered by the extension component according to one or more embodiments of the present disclosure. Additionally or as an alternative, the user interface may further receive an indication whether a user requests that the prediction interval is to be computed according to one or more embodiments of the present disclosure.

**In** a preferred embodiment, the computing device is a portable computing device, including one of a mobile communication device, a smart device, a smart watch, or a personal digital assistant. Preferably, the portable computing device may be carried by a medical profession to estimate probabilities of myocardial infarction for patents.

The current disclosure further defines a system, comprising a server and at least one computing device for estimating myocardial infarction according to any one of the preceding embodiments.

Preferably, the system comprises a server and a computing device for estimating the probability of myocardial infarction, wherein the computing device comprises a receiver configured to receive a set of vital parameters of a subject, the set of vital parameters including troponin data of the subject, the troponin data of the subject being measured using an initial troponin assay a selector configured to select a troponin assay from a set of troponin assays a repository configured to provide a plurality of estimation modules corresponding to the selected troponin assay, wherein the plurality of estimation modules constitute a super learner module, and at least one processor configured to use the plurality of estimation modules to estimate the probability of myocardial infarction of the subject using the set of vital parameters including the troponin data of the subject.

In one embodiment, the system further comprises a network, wherein the at least one computing device is connected to the server via the network, wherein the server is configured to provide the plurality of estimation modules constituting the super learner module to the repository of the at least one computing device. The connection may be a wired or a wireless connection. The plurality of estimation modules may be provided to the computing device when the computing device connects to the server for the first time. The provided plurality of estimation modules may be updated and/or verified at each subsequent connection.

In another embodiment, the system further comprises a database configured to store datasets related to vital parameters including troponin data. The database may be used to estimate the estimation modules constituting the super leaner modules on the respective computing devices. The database may correspond to the database as discussed with regard to one or more embodiments of the present disclosure, such as the BACC database or the stenoCardia database.

According to one embodiment, the server is configured to fit one or more super learner modules with a set of equal weights. A set of equal weights may be estimated for each super learner module. A set of equal weights may be an optimal convex combination for which a score, as computed by a super learner module, is the highest. For example, the score may be the AUC score. The data stored in the database may be used for estimating a set of equal weights for each of the one or more super learner modules. When the one or more super learner modules are each fitted with the set of equal weights, the performance of the fitted super learner modules may be compared with the super learner modules not fitted with the set of equal weights. If a fitted super learner module performs better than a corresponding super learner module not fitted with the set of equal weights, the super learner module fitted with the set of equal weights may be provided to the repository of the computing device. The performance may be compared based on a suitable score, such as the AUC score.

In yet another embodiment, the server is configured to estimate residuals and/or store estimated residuals in one or more pre-computed tables for one or more machines and/or super learner modules. Each of the one more pre-computed tables may be associated with one of the plurality of estimations module constituting one or more super learner modules. The residuals may be estimated according to one or more embodiments of the computing device. The one or more pre-computed tables may also be provided to the at least one computing device when the at least one computing device connects to the server for the first time. The provided one or more pre-computed tables may be updated and/or verified at each subsequent connection.

The current disclosure may further refer to a computer-implemented approach for estimating a probability of myocardial infarction. The approach may comprise performing by a computing device the steps of receiving a set of vital parameters of a subject, the set of vital parameters including troponin data of the subject, the troponin data of the subject being measured using an initial troponin assay, selecting a troponin assay from a set of troponin assays, providing a plurality of estimation modules corresponding to the selected troponin assay, wherein the plurality of estimation modules constitute a super learner module, and using the plurality of estimation modules to estimate the probability of myocardial infarction of the subject using the set of vital parameters including the troponin data of the subject.

It is to be understood that embodiments of the computer-implemented approach may include steps that reflect functional processing of features of embodiments of the computing device or the system, in any combination.

The disclosure further defines one or more computer-readable media storing instructions thereon that when executed on a computing device configure the computing device to perform a method for estimating a probability of myocardial infarction. The computing device may be configured to perform the steps of receiving a set of vital parameters of a subject, the set of vital parameters including troponin data of the subject, the troponin data of the subject being measured using an initial troponin assay, selecting a troponin assay from a set of troponin assays, providing a plurality of estimation modules corresponding to the selected troponin assay, wherein the plurality of estimation modules constitute a super learner module, and using the plurality of estimation modules to estimate the probability of myocardial infarction of the subject using the set of vital parameters including the troponin data of the subject.

It is to be understood that embodiments of the computer-readable media may configure the computing device to perform further steps that reflect embodiments of the computer implemented approach, or functional processing of features of embodiments of the computing device or the system, in any combination.

### BRIEF DESCRIPTION OF THE DRAWINGS

Specific features, aspects and advantages of the present disclosure will be better understood with regard to the following description and accompanying illustrations, where:
- FIG. 1: illustrates a schematic overview of components of a computing device according to one embodiment of the present disclosure;
- FIG. 2: illustrates a flow chart directed at estimation of probabilities of myocardial infarction according to one embodiment of the present disclosure;
- FIG. 3: illustrates a flow chart of a method for estimating machines for a super learner module according to one embodiment of the present disclosure;
- FIGs. 4A to 4D: show scatterplots and corresponding correlations between machines applicable in one embodiment of the present disclosure;
- FIGs. 5A to 5C: illustrate a schematic overview of a user interface suitable for preselecting a troponin assay according to one embodiment of the present disclosure; and
- FIG. 6: illustrates a schematic overview of a system comprising a database, a server and a computing device according to one embodiment of the present disclosure.

### DETAILED DESCRIPTION

In the following description, reference is made to drawings which show by way of illustration various embodiments. Also, various embodiments will be described below by referring to several examples.

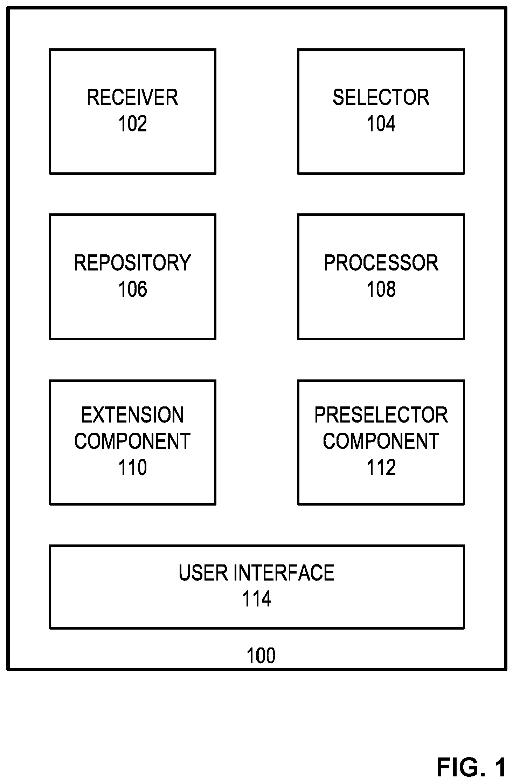

FIG. 1 illustrates a schematic overview of components of a computing device according to one embodiment of the present disclosure. The computing device 100 may be capable of estimating the probability of myocardial infarction. The computing device 100 may include a receiver 102, a selector 104, a repository 106, a processor 108, an extension component 110, a preselector component 112, and a user interface 114. The computing device 100 may be a portable or mobile communication device, such as a smart phone or a tablet.

The receiver 102 may receive a set of vital parameters of a subject, including non-troponin features and troponin data of a subject, which may have been measured using an initial troponin assay. The troponin data may include at least some troponin features of a database of troponin data. The selector 104 may select a troponin assay from a set of troponin assays. The repository 106 may be a storage unit of the computing device 100. The repository 106 may provide an estimation module which corresponds to a selected troponin assay. The repository 106 may store one or more estimation modules. The processor 108 may apply the plurality of estimation modules to estimate a probability of myocardial infarction according to the set of vital parameters of the subject.

The extension component 110 may be used to add additional troponin assays to the set of troponin assays. The extension component 110 may be triggered via a user interaction with the user interface 114 of the computing device 100.

When a set of vital parameters is received by the receiver 102, the preselector component 112 may scan the set of vital parameters in order to estimate a troponin assay that was most likely used for measuring the troponin data in the set of vital parameters. In response to estimating a most likely used troponin marker, the preselector component 112 may preselect a corresponding troponin assay from the set of troponin assays.

The user interface 114 may be connected to the receiver 102, the selector 104, the repository 106, the extension component 110, and the preselector component 112 to provide user input with regard to operation of the respective components and modules. The user interface 114 may further provide the probability of myocardial infarction to the user.

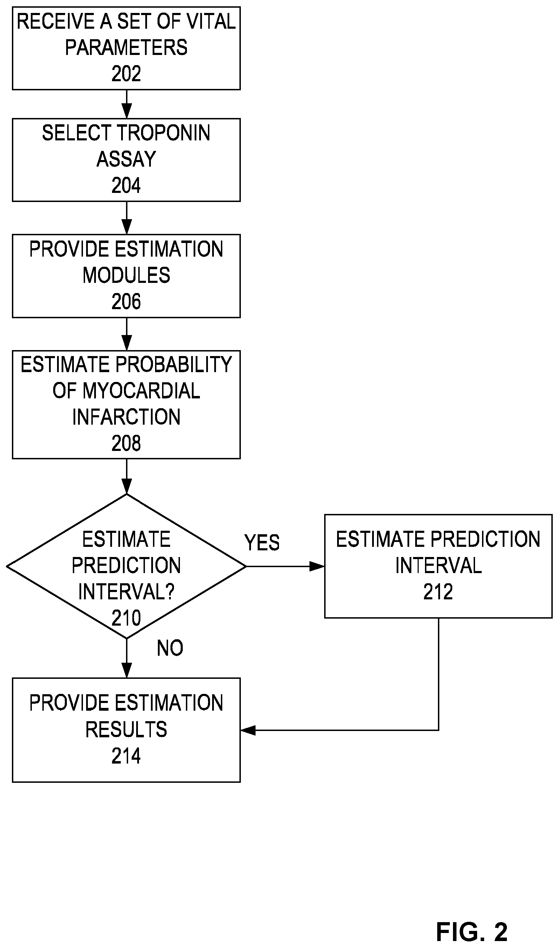

FIG. 2 illustrates a flow chart directed at estimation of probabilities of myocardial infarction according to one embodiment of the present disclosure. The estimation of probabilities may be performed on a computing device, such as the computing device 100 of FIG. 1.

A set of vital parameters may be received by a receiver of the computing device in item 202. The vital parameters may be received by the receiver in response to a request for retrieving the set of vital parameters of a subject. For example, when a patient is delivered to a hospital or any other medical facility, a troponin measurement may be performed using an initial troponin assay. The initial troponin assay may be provided from a manufacturer. The vital parameters of the patient and the troponin measurements may be stored in a local database of the hospital. Before taking further measures for treatment of the patient, the computing device may retrieve the set of vital parameters of the patient from the local database. The set of vital parameters may include non-troponin features and troponin data.

The troponin data may include one or more of information indicating the initial troponin assay, a performed troponin measurement indicating a troponin measurement value of a measured troponin marker, a timestamp of the troponin measurement, and information indicating if the troponin measurement at the timestamp of the troponin measurement was greater than or equal to a LOD. The troponin data may further include a second troponin measurement indicating a second troponin measurement value of a measured troponin marker, a timestamp of the second measurement, and information indicating if the second troponin measurement at the timestamp of the second measurement was greater than or equal to the LOD.

In item 204, a troponin assay may be selected from a set of available troponin assays. A selector of the computing device may estimate whether the selected troponin assay is able to measure the same troponin marker as the initial troponin assay and whether the selected troponin assay and the initial troponin assay are provided from the same manufacturer. The selector may inform and potentially warn a user if a different troponin assay was selected. The selector may further provide a matching score indicating a suitability of the selected troponin assay.

In response to the selection in item 204, the received troponin data of the subject may be scanned. Based on troponin measurements included in the troponin data, a super learner module corresponding to the amount of troponin measurements included in the troponin data may be determined. For example, if the troponin data of the subject comprises an initial troponin measurement, a 1MM of the super learner model may be requested. If the troponin data includes the initial measurement and a second troponin measurement, a 2MM of the super learner model may be requested. The 1MM may comprise one or more machines of differing types, such as a gradient boosting machine, a generalizing additive machine and a generalized boosted regression machine. The 2MM may comprise one or more machines of differing types, such as a generalized additive machine, a generalized boosted regression machine, and a random forest machine.

In item 206, the requested super learner module comprising a plurality of estimation modules corresponding to the respective machines may be provided. The super learner module and its plurality of estimation modules are applied in item 208 to estimate the probability of myocardial infarction of the subject or patient, using the set of vital parameters including the troponin data of the subject or patient as received in item 202.

Subsequent to estimating the probability in item 208, it may be determined in item 210 whether a prediction interval is to be estimated. Either the prediction interval is estimated in item 212 and provided together with the estimated probability in item 214, or the method may directly proceed with item 214 to provide the estimated probability only.

The prediction interval for the estimated probability may be estimated in item 212 by setting a coverage level, drawing equal-sized folds of datasets in at least a part of a database for cross-validation, forming cross-validated observations with prediction variability for each equal-sized fold, determining a mean and standard deviation of the cross-validated observations, and estimating the prediction interval using the coverage level, and the mean and standard deviation of the created observations. The cross-validated observation may be formed by, for each equal-sized fold, training each of the plurality of estimation modules using the equal-sized folds without the respective equal-sized fold, making predictions for all data in the respective equal-sized fold, estimating residuals for all data in the respective equal-sized fold using the predictions, and randomly drawing a number of data without replacement from the respective equal-sized fold, to form the cross-validated observations and a prediction error, using the fold-specific predicted observation and the respective residual of the randomly drawn data.

In one particular example, a starting point for the processing in item 212 related to the formulation of prediction intervals may be *n* independent identically distributed pairs (*yᵢ*, ***x**ᵢ*), where ***x**ᵢ* is a *p* dimensional vector of independent variables and *yᵢ* is a univariate dependent variable. The regression function is $E \left({y}_{i}\left|{x}_{i}\right.\right)$. The aim is to predict a new response *y*₀ from a new observation ***x***₀. This may be termed conformal prediction. Formally, given a coverage level 1 - *α*, the aim is to estimate a prediction interval *C*(***x***₀) based on (*yᵢ*, ***x**ᵢ*), *i* = 1, ... , *n* with the property that $ℙ \left({y}_{0}∈C\left({x}_{0}\right)\right) \geq 1 - α$. The prediction interval may be estimated using cross-validation, according to one or more steps of the following processing structure:
**Step 0:** Set a coverage level 1 - *α* for the prediction interval.
**Step 1:** Draw *V* equal-sized folds for cross-validation, in at least a part of a database comprising with *n* datasets with data pairs (*yᵢ*, ***x**ᵢ*), *i* = 1, ... , *n*. For each fold *v, v =* 1, ... , *V* do:
   - Train each of the plurality of estimation modules using all equal-sized folds but equal-sized fold v: *V*_{(-*v*)}.
   - Make predictions for all *j* = 1, ..., *n*/*V* left-out datasets from the equal-sized fold *v:* ŷ*_{v,j}*(***x**_{v,j}*).
   - Estimate residuals for all left-out datasets: *ε̂_{v,j} = y_{v,j}* - *ŷ_{v,j}*(***x**_{**v**,j}), j* = 1, ... , *n*/*V*.
   - For a new data point ***x***₀ make the prediction *ŷᵥ*(***x***₀).
   - Randomly draw *R* ≤ *n*/*V* subjects without replacement from the left-out datasets of the equal-sized fold v and form future observations with prediction variability: ${\hat{y}}_{v , {r}_{in}}^{∗} = {\hat{y}}_{v} \left({x}_{0}\right) + {\hat{ε}}_{v , {r}_{in}}$, *r*ᵢₙ = 1, ..., *R.*
**Step 2:** Determine the *α*/2 and 1 - *α*/2 quantiles *q_{α}*/2 and *q*₁ -α/2 from the *r V* cross-validated observations ${\hat{y}}_{v , {r}_{in}}^{∗}$.
**Step 3:** The prediction interval *C*(***x***₀) is given by the quantiles obtained in step 2.

As an alternative to Steps 2 and 3, a parametric approach can be used:
**Step 2*:** Determine a mean mean and a standard deviation *SD* of the cross-validated observations ${\hat{y}}_{v , {r}_{in}}^{∗}$. For example, using $mean = \overline{\hat{y}} \left({x}_{0}\right) = \frac{1}{n / V} {\sum }_{j = 1}^{n / V} {\hat{y}}_{v , j} \left({x}_{v , j}\right) and SD = \sqrt{\hat{V} ar \left(\hat{y}*\right) \left({x}_{0}\right)} .$
**Step 3*:** The prediction interval *C*(*x*₀) is calculated as *mean* ± *z*_{1-*α*/2} *SD* with a quantile determined from the standard normal distribution.

In this example, the number *V* of equal sized folds may be, for example, 10. Furthermore, the steps for training each of the plurality of estimation modules, making predictions and estimating residuals may be performed on a server. The estimated residuals *ε̂_{v,j}* = *y_{v,j}* - *ŷ_{v,j}*(***x***_{*v,*j}) may have been stored in one or more pre-computed tables each being associated with an equal-sized fold v and an estimation module. The one or more pre-computed tables may be stored in a repository of the computing device. Thus, the computing device can more efficiently estimate the prediction intervals.

The estimated probability may be further analyzed to estimate a classification result indicating based on the estimated probability if a myocardial infarction can be ruled-out, ruled-in or that a subject may need to be further observed. The estimation may be based on a rule-in cutoff and a rule-out cutoff. The rule-in cutoff and the rule-out cutoff may be stored in the repository and may be associated with the selected troponin assay. The rule-in cutoff and the rule-out cutoff may be associated with either a 1MM or a 2MM. Hence, the rule-in and the rule-out cutoff may be provided based on the amount of troponin measurements included in the troponin data of the subject.

If the estimated probability is greater than the rule-out cutoff and less than or equal to the rule-in cutoff, a clear estimation of a likelihood of myocardial infarction might not be possible. Therefore, the subject may be classified to be observed meaning that additional care and treatments are needed for the subject.

If the estimated probability is less than or equal to the rule-out cutoff, the subject may be ruled-out and therefore classified as not having a myocardial infarction. However, if the estimated probability is greater than the rule-in cutoff, the subject may be ruled-in and therefore classified as likely having a myocardial infarction.

Based on the analysis, a positive predictive value and a negative predictive value may be provided via the user interface of the computing device. Both the negative and the positive predictive value may be stored in the repository of the computing device and may have been estimated on an external server. If the estimated probability was ruled-out, the positive predictive value may be provided via the user interface of the computing device together with the estimated probability of item 208 and the classification result. If a prediction interval was estimated in item 212, the prediction interval may also be provided via the user interface. If the estimated probability was ruled-in, the positive predictive value may be provided via the user interface of the computing device together with the estimated probability of item 208 and the classification result. If a prediction interval was estimated in item 212, the prediction interval may be provided via the user interface.

In one example, ruling-out and ruling-in of acute myocardial infarction (AMI) may be performed according to one or more steps of the following processing structure:
An algorithm $A \left({c}_{1 MM}^{ro}, {c}_{1 MM}^{ri}, {c}_{2 MM}^{ro}, {c}_{2 MM}^{ri}\right)$ may classify patients into one of three categories for classification:
- **Rule-out:** AMI is ruled-out.
- **Rule-in:** AMI is ruled-in.
- **Observe:** AMI can neither be ruled-out nor ruled-in. In this case a patient needs to be observed further (e.g., further tests are required).

Let $0 < {c}_{1 MM}^{ro} \leq {c}_{1 MM}^{ri} < 1$ and $0 < {c}_{2 MM}^{ro} \leq {c}_{2 MM}^{ri} < 1$. These constants are cutoffs that will be used for ruling-out (***ro***) and ruling-in (***ri***) AMI based on the estimated AMI probabilities produced by the 1MM and 2MM of the super learner modules. Cutoffs may vary by troponin assay. Different cutoffs may give different algorithms $A \left({c}_{1 MM}^{ro}, {c}_{1 MM}^{ri}, {c}_{2 MM}^{ro}, {c}_{2 MM}^{ri}\right)$.

During the estimation of the 1MM and 2MM super learner modules, the database related to troponin data, such as the BACC database, may have been divided in disjoint folds of approximately equal sizes. Each patient may belong to a unique fold, and for that fold there are 1MM and 2MM super learner modules that were fitted omitting that fold (and in particular omitting that patient), as described with regard to one or more embodiments and examples. For each patient, the 1MM and 2MM super learner modules fitted on the remaining folds may be used to estimate the AMI probability. Let ***p*_{1*MM*}** and ***p*_{2*MM*}** be the corresponding estimates for the 1MM and 2MM models.

The algorithm $A \left({c}_{1 MM}^{ro}, {c}_{1 MM}^{ri}, {c}_{2 MM}^{ro}, {c}_{2 MM}^{ri}\right)$ may include one or more of the following steps:
**Step 1** (Rule-out 1MM) If ${p}_{1 MM} \leq {c}_{1 MM}^{ro}$, then classify patient as **Rule-out** and go to step 6.
**Step** 2 (Rule-in 1MM) If ${p}_{1 MM} > {c}_{1 MM}^{ri}$, then classify patient as **Rule-in** and go to step 6.
**Step 3** (Rule-out 2MM) If ${p}_{2 MM} \leq {c}_{2 MM}^{ro}$, then classify patient as **Rule-out** and go to step 6.
**Step 4** (Rule-in 1MM) If ${p}_{2 MM} > {c}_{2 MM}^{ri}$, then classify patient as **Rule-in** and go to step 6.
**Step 5** (Observe) Classify patient as **Observe.**
**Step 6** Return classification category of patient.

Algorithm $A \left({c}_{1 MM}^{ro}, {c}_{1 MM}^{ri}, {c}_{2 MM}^{ro}, {c}_{2 MM}^{ri}\right)$ may be applied to all individuals of a database related to troponin data, such as the BACC database.

Sensitivity and specificity of algorithm $A \left({c}_{1 MM}^{ro}, {c}_{1 MM}^{ri}, {c}_{2 MM}^{ro}, {c}_{2 MM}^{ri}\right)$ can be estimated using a plurality of datasets in the database, including all datasets. Sensitivities and specificities for the rule-in and rule-out parts of algorithm $A \left({c}_{1 MM}^{ro}, {c}_{1 MM}^{ri}, {c}_{2 MM}^{ro}, {c}_{2 MM}^{ri}\right)$ can be estimated for a selection of cutoffs and stored. These sensitivities and specificities can be accessed later when applying the algorithm to a new patient (not included in datasets of the database) to determine the positive predictive value (PPV) and the negative predictive value (NPV).

The computation of sensitivity and specificity may require a classification according to two categories. To this end, the rule-out and rule-in parts of the algorithm may be treated separately, and sensitivity and specificity may be estimated for each part. They can be estimated according to one or more of the following steps:
**Step 1** (Sensitivity rule-in part (sensᵣᵢ)) Consider only AMI patients from BACC. Determine proportion of AMI patients fulfilling ${p}_{1 MM} > {c}_{1 MM}^{ri}$ OR $\left({c}_{1 MM}^{ro}<{p}_{1 MM}\leq {c}_{1 MM}^{ri} AND {p}_{2 MM}>{c}_{2 MM}^{ri}\right)$ . This proportion is the sensitivity of the rule-in part.
**Step 2** (Specificity rule-in part (specᵣᵢ)) Consider only patients without MI from BACC. Determine proportion of patients without AMI fulfilling ${p}_{1 MM} > {c}_{1 MM}^{ri}$ OR $\left({c}_{1 MM}^{ro}<{p}_{1 MM}\leq {c}_{1 MM}^{ri} AND {p}_{2 MM}>{c}_{2 MM}^{ri}\right)$. Specificity rule-in part equals 1 minus this proportion.
**Step 3** (Specificity rule-out part (specᵣₒ)) Consider only patients without AMI from BACC. Determine proportion of patients without AMI fulfilling ${p}_{1 MM} \leq {c}_{1 MM}^{ro}$ OR $\left({c}_{1 MM}^{ro}<{p}_{1 MM}\leq {c}_{1 MM}^{ri} AND {p}_{2 MM}\leq {c}_{2 MM}^{ro}\right)$. This proportion is the specificity of the rule-out part.
**Step 4** (Sensitivity rule-out part (sensᵣₒ)) Consider only AMI patients from BACC. Determine proportion of AMI patients fulfilling ${p}_{1 MM} \leq {c}_{1 MM}^{ro} OR \left({c}_{1 MM}^{ro}<{p}_{1 MM}\leq {c}_{1 MM}^{ri} AND {p}_{2 MM}\leq {c}_{2 MM}^{ro}\right)$ . Sensitivity rule-out part equals 1 minus this proportion.

A positive predictive value (PPV) can only be calculated for patients who are ruled in (meaning that they are classified as AMI patients by the super learner module).

A negative predictive value (NPV) can only be calculated for patients who are ruled out (meaning that they are classified as no AMI patients by the super learner module).

The calculation of NPV and PPV requires an additional new parameter, the prevalence of AMI ***Prev_{ANI}.*** This is a probability, thus **0** *< **Prev_{AMI}** <* **1.**

The formulae for estimating PPV and NPV are as follows:
Step 1 $PPV = \frac{sen {s}_{ri} ⋅ Pre {v}_{AMI}}{sen {s}_{ri} ⋅ Pre {v}_{AMI} + \left(1-spe{c}_{ri}\right) ⋅ \left(1-Pre{v}_{AMI}\right)}$
Step 2 $NPV = \frac{spe {c}_{ro} ⋅ \left(1-Pre{v}_{AMI}\right)}{spe {c}_{ro} ⋅ \left(1-Pre{v}_{AMI}\right) + \left(1-sen{s}_{ro}\right) ⋅ Pre {v}_{AMI}} .$

The PPV from step 1 for a new patient ruled in as AMI may be displayed or otherwise provided via the user interface. The NPV from step 2 may be displayed or otherwise provided via the user interface. Additionally the NPV may be provided together with a message, such as "if the patient had been ruled out from AMI, he/she would have had an NPV of ...", thereby indicating the value.

Similarly, the NPV from step 2 for a new patient ruled out from AMI may be displayed or otherwise provided via the user interface. This may include the PPV from step 1, preferably with a message, such as "if the patient had been ruled it for AMI, he/she would have had a PPV of ...", thereby indicating the value.

According to one example, at least one of the plurality of estimation modules may comprise an estimation module based on a random forest. The random forest may comprise data related to an associated troponin assay. The random forest may comprise a plurality of decision trees. A decision tree may be created by deriving a bootstrapped dataset, selecting a random subset of variables, retrieving partitions from the bootstrapped dataset for each query associated with each variable in the subset of variables, assigning a partition to a node, assigning the query used for retrieving the partition to the node, erasing data corresponding to the assigned partition from the bootstrapped dataset, and either adding at least two edges to the node or adding no edges to the node. A random forest may comprise any number of decision trees to enable a reliable estimation of probabilities. As an example, the random forest may include at least 1000 decision trees. A random forest may comprise datasets from the database. Deriving the bootstrapped dataset may comprise randomly drawing datasets associated with the troponin assay. The datasets for the bootstrapped dataset may be drawn randomly with reputation from the database. Each dataset of the randomly drawn data may be included one or more times in the bootstrapped dataset. The bootstrapped dataset may comprise any suitable number of datasets. A suitable number of datasets may preferably be at least 500, 600, 700 or 800 datasets, and most preferably, 1000 datasets. However, in some examples, the suitable number of datasets may also be less than 500 or even more than 1000. In other examples, a suitable number of datasets may also be half of the datasets in the database.

A decision tree may be created in a recursive manner. If a decision tree does not include any of the nodes, a root node may be created. A random subset of variables may be selected randomly from a subset of variables. Each variable of the subset of variables may include one or more queries associated with the variable. The variables of the subset of variables may correspond to non-troponin features and troponin features of the database. The one or more queries associated with each variable of the subset of variables may be associated to values of the non-troponin features and troponin features. For each variable from the random subset of variables, one or more partitions of the bootstrapped dataset may be retrieved. Each retrieved partition may be associated with a query associated with a variable of the random subset of variables.

Assigning a partition as a node may include estimating a probability of myocardial infarction for each partition. A probability of myocardial infarction for a partition may be a relative portion of datasets in the partition whose myocardial features indicates the presence of myocardial infarction. The presence of myocardial infarction may be indicated if a value of a myocardial feature is true. Based on the estimated probabilities for each partition, a mean average value may be computed. Based on the mean average value, mean squared errors with respect to the mean average value may be computed for each estimated probability of each partition. The partition having the least mean squared error of the computed mean squared error may be assigned to the node. The query used to query the partition may also be assigned to the node.

When a partition is assigned to a node, datasets corresponding to the assigned partition may be erased from the bootstrapped dataset. Erasing data from the bootstrapped dataset may correspond to removing datasets that correspond to the partition from the bootstrapped dataset. If the amount of datasets of the partition assigned to the node is greater than a threshold, at least two edges may be added to the node. The threshold may represent a relative portion of datasets in a partition in relation to the datasets of the derived bootstrapped dataset. The relative portion may be at least 0.01 to 0.2. Preferably, the relative portion may be 0.1. However, in other examples, the relative portion may be 0.05 or any other suitable fraction. If the amount of datasets of the partition assigned to the node is less than or equal to the threshold, no edges may be added to the node.

The at least two edges may represent a decision outcome based on the query assigned to the node. One of the at least two edges may be labeled with a true label and one of the at least two edges may be labeled with a false label. A node may be created and assigned to each of the at least two edges. For each created and assigned node, selecting a random subset of variables, retrieving partitions, assigning a partition to a node, assigned the query used for retrieving the partition, erasing data corresponding to the assigned partition, and adding at least two edges, or adding no edges may be repeated using the derived bootstrapped dataset, wherein the assigned partition may be erased from the bootstrapped dataset.

Using an estimation module that comprises a random forest may include inputting the set of vital parameters into the random forest, wherein the set of vital parameters walk through each decision tree of the random forest. The set of vital parameters may be inputted into each decision tree of the random forest. The set of vital parameters may be inputted into the root node of each decision tree. The at least one processor may iteratively select each decision tree one after another and input the set of vital parameters into the root node of each decision tree.

Walking through each decision tree of the random forest may include querying the set of vital parameter at each node of the decision tree. Querying may include querying the set of vital parameters with the query assigned to the node. Querying may further include estimating a decision outcome. The decision outcome may be estimating if the query assigned to the node is applicable to the set of vital parameters. The assigned node may be applicable to the set of vital parameters if information corresponding to the query may be retrieved from the set of vital parameters. Based on the decision outcome, the set of vital parameters may walk along one of the at least two edges of the node to a next node. The next node may be a node assigned to one of the at least two edges. If the assigned query is applicable to the set of vital parameters, the decision outcome may be true. If the decision outcome is true, the set of vital parameters may walk along the edge labeled with the true label. If the assigned query is not applicable to the set of vital parameters, the decision outcome may be false. If the decision outcome is false, the set of vital parameters may walk along the edge labeled with the false label. If the next node also includes at least two edges, walking through the decision tree is repeated with the next node. If the next node does not include at least two edges, walking through the decision tree may end.

When said walking through the decision tree ends at a node, a probability of myocardial infarction of a decision tree is a percentage of subjects having a myocardial infarction of a partition assigned to the node. The probability may be a relative portion of datasets in the partition whose myocardial features indicates the presence of myocardial infarction. The presence of myocardial infarction may be indicated if a value of a myocardial feature is yes.

Estimating the probability of myocardial infarction for the at least one estimation module that comprises a random forest may include retrieving a plurality of probabilities for myocardial infarctions from the plurality of estimation modules and computing an average value based on the retrieved plurality of probabilities. Preferably, a probability of myocardial infarction is retrieved from each decision tree of a random forest of the plurality of estimation modules. The computed average value may be the probability of myocardial infarction for the subject based on the set of vital parameters.

It is to be understood that an estimation module based on random forest is an example only. Other machines for estimating probabilities are encompassed as well and described in detail with regard to examples and embodiments of the present disclosure.

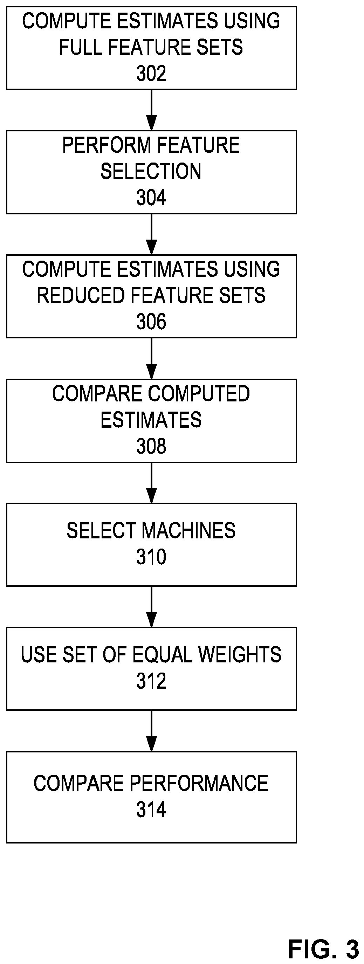

FIG. 3 illustrates a flow chart of a method for estimating machines for a super learner module according to one embodiment of the present disclosure. The method may be performed on a server.

A database related to troponin measurements may be used as inputs for estimating the performances of different types of machines that may be selected for the super learner module. For example, the BACC database may be used. However, it is to be understood that any suitable database with troponin data and non-troponin data related to troponin measurements may be provided as a basis for estimating performance of machines. In this example embodiment, machines for a 1MM, as discussed with regard to embodiments of the present disclosure, may be selected. This method may also be applied for estimating machines that may be selected for a 2MM, as discussed with regard to embodiments of the present disclosure. It is to be understood, that machines estimated for the 2MM may differ from machines estimated for the 1MM.

When estimating machines for the 1MM, full features may comprise non-troponin features, troponin features with only initial troponin measurements and myocardial features of the datasets of the database. When estimating machines for the 2MM, full features may be non-troponin features, troponin features with initial troponin measurements and second troponin measurements, and myocardial features of datasets of the database. The full features may comprise full features sets. Each full feature set may be associated with one troponin assay included in the database. In one example, the troponin assays included in the database may be assay 1, assay 2, assay 3 and assay 4. Therefore, one full feature set may only include datasets associated with assay 1, one full feature set may only include datasets associated with assay 2, one full feature set may only include datasets associated with assay 3, and one full feature set may only include datasets associated with assay 4. However, it is to be understood that four troponin assays are an example only and more or less troponin assays can be recorded in the database, such as 2, 3, 5, 6, 7, or 8 troponin assays, and the present disclosure is not limited by a certain number of troponin assays.

In item 302, performance estimates using full features may be computed. Computing a performance estimate for one troponin assay for one machine may correspond to computing a performance estimate using a full feature set associated with one troponin assay and using one machine. In a particular example used for illustrative purposed, each full feature set may be divided into, for example, 10 equal-sized partitions. For a full feature set associated with one troponin assay, each machine from the set of machines may be fitted with, for example, 9 of the 10 partitions. This is for illustrative purposes only. After fitting a machine, a performance of the machine may be estimated by using at least one remaining partition not used for fitting the machine. The remaining partition may be used to estimate the performance of the machines multiple times, such as 10 times. In each performance estimate an AUC score and Brier score based on the AUC score may be obtained. The average of the 10 obtained estimates may then be used to estimate a performance of one machine for one troponin assay included in the database.

Each machine from the set of machines may estimate AUC scores and Brier scores in a different manner. When using the logistic regression machine (lr), all features may be inputted into the machine and modelled into a linear form. When using the logistic regression machine with backward elimination (lrbs), candidate features may be modeled linearly and backward elimination may be performed. This procedure may begin with a model that includes all candidate features, wherein candidate features may be dropped whose significant level may below ***α*.** This may be repeated until no non-significant candidate features remain. The significance level ***α*** for lrbs may be set to 0.05. When using the logistic regression machine with univariate screening (lrus) may compute a regression for each feature. Herein, all regressions may be evaluated and only the features whose p-value may be less than 0.05 may be included into a final univariate model used for estimating AUC scores and Brier scores. The component-wise gradient boosting machine (glmboost) may use univariate logistic regressions as a base learner. Herein, the number of boosting iterations may be tuned using 10 randomly drawn partitions from the database, wherein a maximum number of boosting iterations may be set to 10000. The generalized additive model (gam) may use logistic regression with the continuous predic-tors modeled in a flexible manner using smoothers. The elastic net logistic regression machine (en) may use a mixture of a ridge (***L*₂** penalization) and a lasso (***L*₁** penalization). Herein, when the elastic net shrinks, the candidate features towards zero and according to the lasso feature may be selected. The elastic net mixing parameter and the penalization parameter may be estimated by 10 randomly drawn partitions from the database. When using multivariate adaptive regression splines (mars), 10 randomly drawn partitions from the database may be used for selecting a maximum degree of interactions and a maximum number of terms in the model. For gradient boosting machine (gbm) a learning rate may be set to 0.01 and a maximum tree depth to 2. A number of boosting iterations may be tuned using 10 randomly drawn partitions from the database, wherein the maximum number of boosting iterations may be 10000. The random forest machine (rf) may be in regression mode using subsampling and may select rank statistics as a split rule. The random forest machine with feature selection (rffs) may be in regression mode and use subsampling with maximally selected rank statistics as split rules, wherein features may be kept if a p-value is lower than 0.05. The support vector machine (svm) may use a radial basis function kernel. 10 randomly drawn partitions from the database may be used to tune a radial basis function parameter γ and a cost (penalization) parameter.

Feature selection is performed in item 304 in order to reduce the feature space and amount of datasets of the full features. The steps for selecting one or more features may include selecting features and values corresponding to age and gender features and selecting one or more features and a corresponding values that may be included at least five times in at least one partition of the full feature sets of the full features. For example, if the feature and the corresponding feature diabetes and the corresponding value yes is included in one partition five times, the feature diabetes yes may be selected to the selected features. Features selected this way may be applied when performing feature selection for all partitions of the full feature sets of the full features.

If selected, values corresponding to eGFR features may be discarded. Discarding eGFR features may achieve a simpler feature set for computation because of a high correlation between the features and values corresponding to age and eGFR. The correlation may be around -0.6. If one or more troponin measurement values were selected, LODs corresponding to the one or more troponin measurement values may be selected. Moreover, if one or more LODs and corresponding values were selected, troponin measurement values corresponding to the selected LODs may be selected.

The feature selection is performed on all full feature sets of the full features in order to create reduced feature by reducing amount of datasets and feature space of the full features. For each full features set of the full features, features selection may be performed by retrieving datasets from the full feature set include at least one feature and a corresponding value that corresponds to one feature and a corresponding value of the one or more selected features. Datasets retrieved for a full feature set may be a reduced feature set. The reduced features may be all retrieved datasets for each full feature set. Therefore, one reduced feature set may only include datasets associated with assay 1, one reduced feature set may only include datasets associated with assay 2, one reduced feature set may only include datasets associated with assay 3, and one reduced feature set may only include datasets associated with assay 4. In this example, each reduced feature set may be divided into 10 partitions. It is to be understood that any ranges or numbers as indicated in this exemplifying embodiment are for illustrative purposes only. Other values can be used as well.

In item 306, the performance estimates may be computed using the reduced features, the logistic regression machine with backward elimination, logistic regression machine with univariate screening and the random forest machine with feature selection from the set of machines may not be used, since these machines perform intrinsic feature selection. Computing performance estimates using reduced feature in item 306 may correspond to computing performance estimates using full features in item 302.

The computed performance estimates between the machines using the full features and the machines using the reduced features may be compared in item 308.

The following tables show a performance comparison between computed performance estimates of a full and reduced feature sets for troponin assays included in the BACC database, as an example.

**Table 1: Computed performance estimates of full and reduced feature sets using troponin assay 1 of the BACC database.**

| Full feature machines | Full features AUC scores | Reduced feature machines | Reduced features AUC scores | Full feature machines | Full features Brier scores | Reduced feature machines | Reduced features Brier scores |
|---|---|---|---|---|---|---|---|
| gbm | 0.91769 | glmboost | 0.91523 | gam | 0.07844 | glmboost | 0.7979 |
| gam | 0.91674 | gam | 0.91492 | glmboost | 0.07881 | en | 0.07982 |
| en | 0.91535 | lr | 0.91485 | en | 0.07886 | lr | 0.07987 |
| glmboost | 0.91505 | en | 0.91471 | gbm | 0.079 | mars | 0.08018 |
| lr | 0.91356 | mars | 0.91451 | lr | 0.07904 | gbm | 0.08027 |
| lrbs | 0.91315 | gbm | 0.914 | lrbs | 0.07949 | gam | 0.0804 |
| rf | 0.91311 | rf | 0.91056 | svm | 0.08053 | svm | 0.08412 |
| mars | 0.90768 | svm | 0.99508 | lrus | 0.08174 | rf | 0.09006 |
| lrus | 0.90532 | | | mars | 0.08217 | | |
| rffs | 0.90512 | | | rf | 0.08973 | | |
| svm | 0.9023 | | | rffs | 0.0916 | | |

**Table 2: Computed performance estimates of the full and reduced feature sets using troponin assay 2 of the BACC database.**

| Full feature machines | Full features AUC scores | Reduced feature machines | Reduced features AUC scores | Full feature machines | Full features Brier scores | Reduced feature machines | Reduced features Brier scores |
|---|---|---|---|---|---|---|---|
| gam | 0.91443 | glmboost | 0.90716 | gam | 0.07917 | glmboost | 0.08399 |
| gbm | 0.91372 | en | 0.90684 | gbm | 0.07999 | lr | 0.08409 |
| glmboost | 0.90799 | gbm | 0.9067 | svm | 0.08022 | en | 0.0841 |
| en | 0.9074 | lr | 0.9063 | lr | 0.08053 | gam | 0.08438 |
| lrbs | 0.90726 | gam | 0.9061 | glmboost | 0.08054 | gbm | 0.08521 |
| lr | 0.90643 | mars | 0.90067 | en | 0.08086 | mars | 0.08535 |
| lrus | 0.89822 | rf | 0.89449 | lrbs | 0.08134 | svm | 0.08977 |
| mars | 0.89806 | svm | 0.85141 | mars | 0.0838 | rf | 0.09367 |
| svm | 0.89756 | | | lrus | 0.08395 | | |
| rf | 0.89601 | | | rffs | 0.0918 | | |
| rffs | 0.8839 | | | rf | 0.09303 | | |

**Table 3: Computed performance estimates of the full and reduced feature sets using troponin assay 3 of the BACC database.**

| Full feature machines | Full features AUC scores | Reduced feature machines | Reduced features AUC scores | Full feature machines | Full features Brier scores | Reduced feature machines | Reduced features Brier scores |
|---|---|---|---|---|---|---|---|
| gam | 0.92514 | gam | 0.92318 | gam | 0.07665 | gam | 0.07839 |
| gbm | 0.92467 | glmboost | 0.92197 | mars | 0.07672 | glmboost | 0.0785 |
| mars | 0.92362 | en | 0.92159 | en | 0.07725 | en | 0.07855 |
| en | 0.92279 | lr | 0.92158 | glmboost | 0.0774 | lr | 0.07859 |
| glmboost | 0.92238 | mars | 0.92024 | gbm | 0.0774 | mars | 0.07911 |
| lr | 0.92097 | gbm | 0.9199 | lr | 0.0777 | gbm | 0.07912 |
| lrbs | 0.91985 | rf | 0.91583 | lrbs | 0.07792 | svm | 0.08761 |
| rf | 0.91628 | svm | 0.87544 | svm | 0.07935 | rf | 0.09051 |
| lrus | 0.91311 | | | lrus | 0.08069 | | |
| rffs | 0.90969 | | | rf | 0.09001 | | |
| svm | 0.90573 | | | rffs | 0.09119 | | |

**Table 4: Computed performance estimates of the full and reduced feature sets using troponin assay 4 of the BACC database.**

| Full feature machines | Full features AUC scores | Reduced feature machines | Reduced features AUC scores | Full feature machines | Full features Brier scores | Reduced feature machines | Reduced features Brier scores |
|---|---|---|---|---|---|---|---|
| gam | 0.92968 | gam | 0.9271 | gam | 0.07781 | gam | 0.0784 |
| gbm | 0.92964 | gbm | 0.92494 | en | 0.07874 | en | 0.07926 |
| lrbs | 0.92404 | glmboost | 0.92328 | glmboost | 0.07881 | glmboost | 0.07932 |
| glmboost | 0.9231 | lr | 0.92307 | gbm | 0.07891 | lr | 0.0794 |
| en | 0.92247 | en | 0.92294 | lrbs | 0.07912 | gbm | 0.08053 |
| lr | 0.92071 | rf | 0.91849 | lr | 0.07937 | mars | 0.08352 |
| rf | 0.91845 | mars | 0.9127 | svm | 0.08119 | svm | 0.08444 |
| mars | 0.91578 | svm | 0.87492 | lrus | 0.08174 | rf | 0.0905 |
| lrus | 0.91366 | | | mars | 0.08315 | | |
| svm | 0.91115 | | | rffs | 0.09116 | | |
| rffs | 0.91019 | | | rf | 0.09204 | | |

In the tables 1 to 4, the columns "Full feature machines" and "Reduced feature machines" represent the machines used for computing performances estimates using the full features and the reduced features respectively. The columns "Full features AUC scores" and "Full features Brier scores" represent the AUC scores and Brier scores each machine using the full features computed. The columns "Reduced features AUC scores" and "Reduced features Brier scores" represent the AUC scores and Brier scores each machine using the reduced features computed. AUC scores are sorted in a descending order starting from the highest computed AUC score for a machine. Brier scores are sorted in an ascending order starting from the lowest computed Brier score for a machine.

Table 1 illustrates results of the computed performance estimates for troponin assay 1, table 2 illustrates results of the computed performance estimates for troponin assay 2, table 3 illustrates results of the computed performance estimates for troponin assay 3, and table 4 illustrates results of the computed performance estimates for troponin assay 4.

As shown in the tables 1 to 4, based on comparing the computed estimation performances between the machines using full features and machines using reduced features, machines using the full features set achieved slightly higher AUC scores and lower Brier scores than the machines using the reduced feature sets. Yet, for further evaluation, the computed performance estimates of the reduced feature may be further reviewed, since the reduced features may be considered to only contain features and corresponding values of higher relevancy for myocardial infarctions. Therefore, the additional steps for estimating the machines for the super learner module may be performed using the reduced features.

The performances between the machines using the reduced features may be further compared in item 308 by ranking the machines based on the computed AUC scores and Brier scores for each assay in an automated manner. Rankings for the computed performance estimates of the example, as indicated in the tables above, based on the reduced features for the AUC scores and for the Brier scores are shown in the subsequent tables.

**Table 5: Ranking of the machines using the reduced features based on the computed AUC scores.**

| Machine | Assay 1 ranking | Assay 2 ranking | Assay 3 ranking | Assay 4 ranking | Summed ranks |
|---|---|---|---|---|---|
| glmboost | 1 | 1 | 2 | 3 | 7 |
| gam | 2 | 5 | 1 | 1 | 9 |
| en | 4 | 2 | 3 | 5 | 14 |
| lr | 3 | 4 | 4 | 4 | 15 |
| gbm | 6 | 3 | 6 | 2 | 17 |
| mars | 5 | 6 | 5 | 7 | 23 |
| rf | 7 | 7 | 7 | 6 | 27 |
| svm | 8 | 8 | 8 | 8 | 32 |

**Table 6: Ranking of the machines using the reduced features based on the computed AUC scores.**

| Machine | Assay 1 ranking | Assay 2 ranking | Assay 3 ranking | Assay 4 ranking | Summed ranks |
|---|---|---|---|---|---|
| glmboost | 1 | 1 | 2 | 3 | 7 |
| en | 2 | 3 | 3 | 2 | 10 |
| gam | 6 | 4 | 1 | 1 | 12 |
| lr | 3 | 2 | 4 | 4 | 13 |
| gbm | 5 | 5 | 6 | 5 | 21 |
| mars | 4 | 6 | 5 | 6 | 21 |
| svm | 7 | 7 | 7 | 7 | 28 |
| rf | 8 | 8 | 8 | 8 | 32 |

The performances for the reduced features achieved by each machine for each assay are listed in tables 1, 2, 3 and 4. The best performance is ranked with a 1 and the subsequent best performances are ranked with 2, 3, 4, etc. The assigned ranks of each machine for each assay may be summed up and the machine with the lowest rank is considered as the best performing machine.

Based on table 5 relating to AUC scores, the component-wise gradient boosting machine (glmboost) achieved the highest AUC score for assay 1 and assay 2, the second best performance for assay 3, and the third best result for assay 4. When summing up the ranks assigned to glmboost and comparing with the other summed ranks of the other machines, glmboost has the rank with the lowest value. Therefore, glmboost may be considered as the best performing machine, when computing AUC scores using the reduced feature set. In this example, the second best machine is the generalized additive model machines (gam), the third performing machines is the logistic net regression machines (en) and so on. The following best machines based on the computed AUC scores can be viewed in the summed ranks column of table 5.

Based on table 6 relating to Brier scores, the machine glmboost was also ranked as the best performing machine when analyzing estimated Brier scores. When ranking the Brier scores, the lowest value is considered as the best result. In this example, glmboost achieved the lowest Brier Score for the assays 1 and 2, the second lowest for assay 3, and the third best result for assay 4. When summing up the assigned ranks of glmboost and comparing with the other summed ranks, glmboost has the rank with the lowest value. Therefore, glmboost is again considered as the best performing machine, when analyzing computed Brier scores using the reduced feature set. The following best machines based on the computed Brier scores can be viewed in the summed ranks column of table 6.

At this stage, it may be unclear which machines are the best performing machines, since rankings between the AUC scores and Brier scores for certain machines may differ from each other. For example, when viewing the AUC score ranking in the respective table, gam is the second best performing machine and en is the third best performing machine. However, when reviewing the Brier score ranking in the respective table ranking en is the second best performing machine and gam is the third best performing machine. Therefore, in an additional step, correlations and corresponding scatterplots between the performances of the machines may be analyzed. However, since glmboost is ranked as the best machine for both AUC scores and Brier scores, glmboost may be selected as a machine for the super learner module. Based on the subsequent ranked machines of the AUC score ranking as illustrated in the respective table, two more machines may be selected by analyzing scatterplots and correlations between glmboost and a subsequent following machine. When computing scatterplots and correlations between glmboost and subsequent following machine for each assay, the remaining partition of each reduce feature set, which was not used to fit the machines, may be inputted into glmboost and the subsequent following machine.

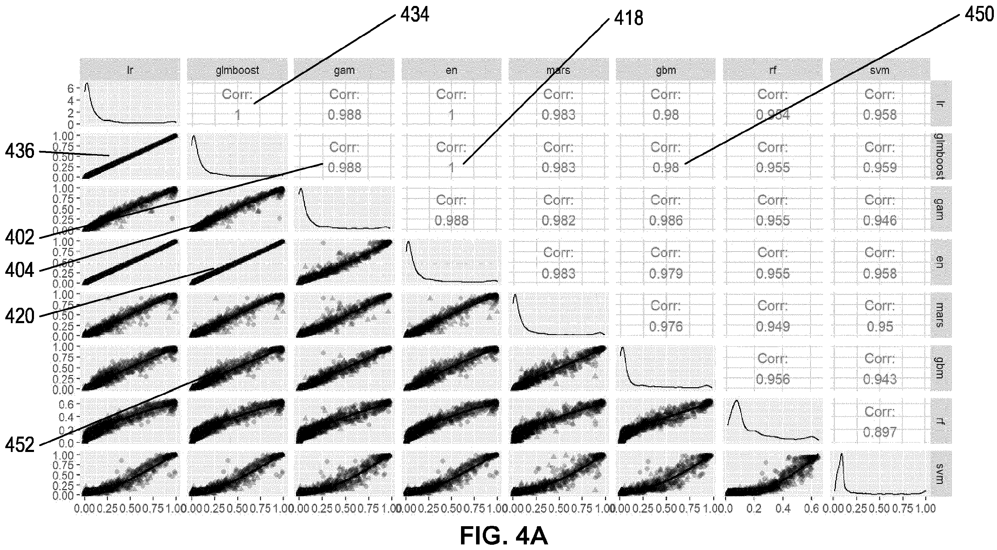

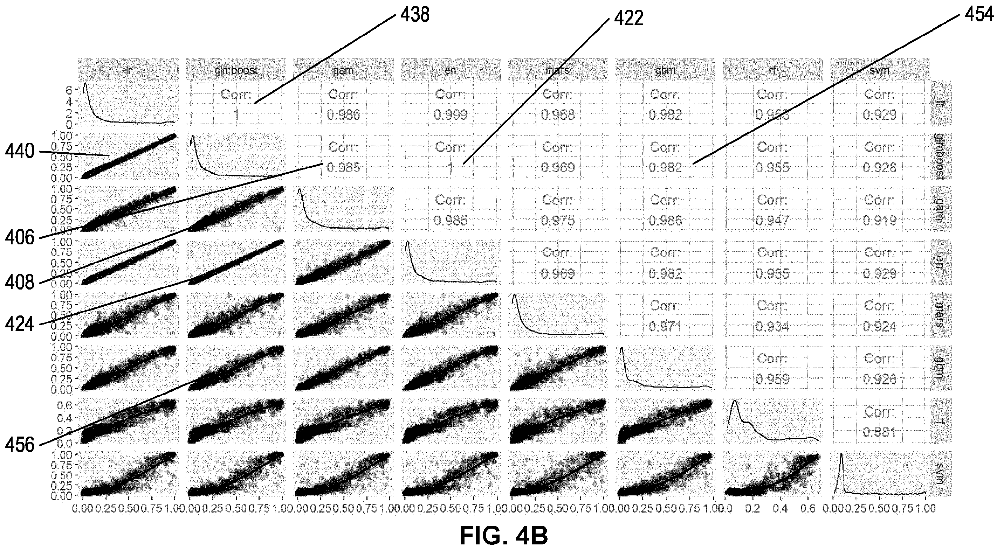

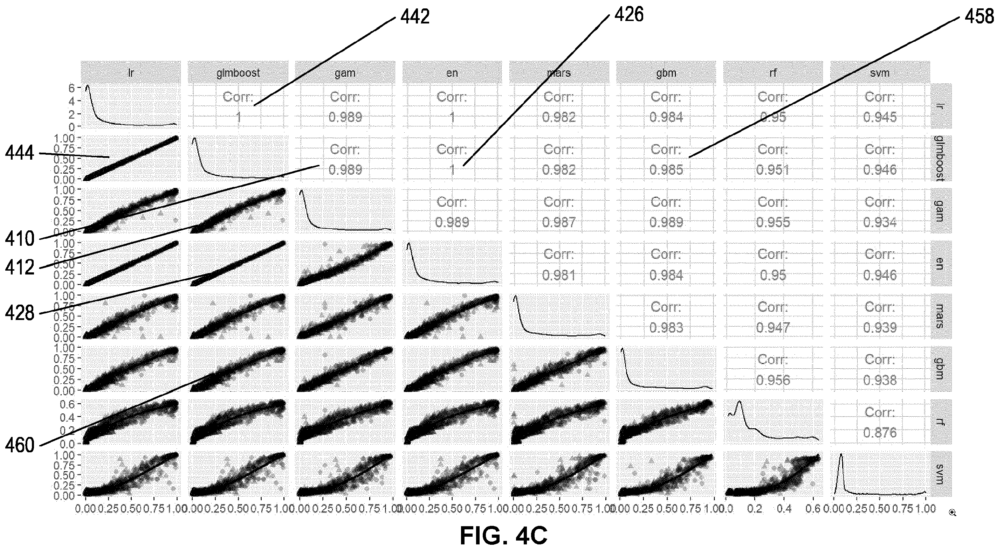

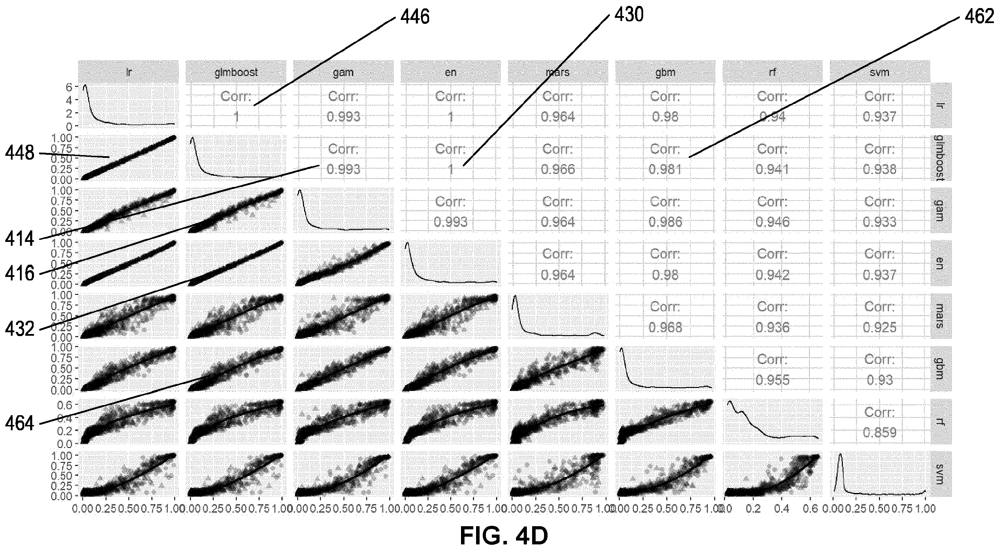

FIGs. 4A to 4D show scatterplots and corresponding correlations between machines applicable in one embodiment of the present disclosure. This may relate to troponin assays included in the BACC. The remaining partition of each reduced feature set may be inputted into glmboost and a subsequent following machine based on the AUC score ranking. Since gam is the second best performing machine when reviewing the AUC scores, scatterplots and a correlation may be analyzed for each assay included in the BACC. When reviewing the correlation between gam and glmboost 402 for troponin assay 1 as illustrated in FIG. 4A, the correlations is not 1. The corresponding scatterplot 404 also does not form an ideal graph. When reviewing the correlations between gam and glmboost for assay 2 406, assay 3 410 and assay 4 414 as illustrated in the FIGs. 4B, 4C and 4D, respectively, the correlations are also not 1. Moreover, the corresponding scatterplots also do not form an ideal graph for troponin assay 2 408, troponin assay 3 412 and troponin assay 4 416. Therefore, gam may be automatically selected as a machine for the super learner module.

The next subsequent following machine, based on the AUC score ranking table 5 may be en. When reviewing the correlation between en and glmboost 418 for troponin assay 1 as illustrated in figure 4A, the correlation is 1. The corresponding scatterplot 420 also forms an ideal graph. When reviewing the correlations between en and glmboost for troponin assay 2 422, troponin assay 3 426, and troponin assay 4 430 as illustrated in the FIGs. 4B, 4C and 4D respectively, the correlations are also 1. Moreover, the corresponding scatterplots also form ideal graphs for assay 2 424, assay 3 428 and assay 4 432 respectively. Therefore, en may not be selected as a machine for the super learner module.

The next subsequent following machine, based on the AUC score ranking in table 5 following en may be lr. When reviewing the correlation between lr and glmboost 434 for troponin assay 1 as illustrated in FIG. 4A, the correlation is 1. The corresponding scatterplot 436 also forms an ideal graph. When reviewing the correlations between lr and glmboost for troponin assay 2 438, troponin assay 3 442 and troponin assay 4 446 as illustrated in the FIGs. 4B, 4C and 4D, respectively, the correlations are also 1 Moreover, the corresponding scatterplots also form ideal graphs for troponin assay 2 440, troponin assay 3 444 and troponin assay 4 448 respectively. Therefore, lr may not be selected as a machine for the super learner module.

The next subsequent following machine, based on the AUC score ranking in table 5 following lr may be gbm. When reviewing the correlation between gbm and glmboost 450 for troponin assay 1 as illustrated in FIG. 4A, the correlations is not 1. The corresponding scatterplot 452 also does not form an ideal graph. When reviewing the correlations between gbm and glmboost for troponin assay 2 454, troponin assay 3 458 and troponin assay 4 462 as illustrated in the FIGs. 4B, 4C and 4D, respectively, the correlations are also not 1. Moreover, the corresponding scatterplots also do not form an ideal graph for troponin assay 2 456, troponin assay 3 460 and troponin assay 4 464. Therefore, gbm may be automatically selected as a machine for the super learner module. Therefore, glmboost, gam and gbm may be selected 310 as the machines for the super learner module, which may be reflected in respective estimation modules.

In another example, when estimating machines for the 2 measurement module, gam, gbm and rf may be estimated as the machines for the super learner module.

This approach may be fully automated based on comparison of the presented parameters, such as estimates of correlations, and/or determination, whether scatterplots form an ideal graph, thereby enabling a fully automated estimation of most suitable machines for the super learner module.

Referring back to FIG. 3, a set of equal weights may be estimated in item 312 in order to combine the selected machines. The set of equal weights may be considered as an optimal convex combination for the machines, which may be estimated via a grid-search with grid width 0.005 from 0 to 1. The database may be divided into 10 randomly drawn partitions, for example. Again, these values and ranges are for illustrative purposes as chosen in one exemplifying embodiment for the BACC database, and it is to be understood that other values and ranges are encompassed by the present disclosure. The set of equal weights may be an optimal convex combination for which the machines glmboost, gam and gbm selected for the 1MM for which the highest average AUC score. The average AUC score may be based on AUC scores each machine of the super learner module may estimate.

The performance between the super learner module, the super learner module with equal weights, and each machine may be compared in item 314 in order to verify if the super learner with equal weights is the best performing machine. The reduced feature sets to may be used to estimate AUC scores and Brier scores, as shown in subsequent tables for example assay 1 to assay 4, respectively. This is reflected in subsequent tables:

**Table 7: Computed performances using assay 1 for the super learner with equal weights (slew), the super learner module (sl), and the set of machines used for the reduced features.**

| Reduced machine | Reduced machine AUC | Reduced machine | Reduced machine BS |
|---|---|---|---|
| slew | 0.91673 | slew | 0.07947 |
| sl | 0.91638 | sl | 0.0795 |
| glmboost | 0.91523 | glmboost | 0.07979 |
| gam | 0.91492 | en | 0.07982 |
| lr | 0.91485 | lr | 0.07987 |
| en | 0.91471 | mars | 0.08018 |
| mars | 0.91451 | gbm | 0.08027 |
| gbm | 0.914 | gam | 0.0804 |
| rf | 0.91056 | svm | 0.08412 |
| svm | 0.88508 | rf | 0.09006 |

**Table 8: Computed performances using assay 2 for the super learner with equal weights (slew), the super learner module (sl), and the set of machines used for the reduced features.**

| Reduced machine | Reduced machine AUC | Reduced machine | Reduced machine BS |
|---|---|---|---|
| sl | 0.90987 | slew | 0.08386 |
| slew | 0.90986 | sl | 0.08395 |
| glmboost | 0.90716 | glmboost | 0.08399 |
| en | 0.90684 | lr | 0.08409 |
| gbm | 0.9067 | en | 0.0841 |
| lr | 0.9063 | gam | 0.08438 |
| gam | 0.9061 | gbm | 0.08521 |
| mars | 0.90067 | mars | 0.08535 |
| rf | 0.89449 | svm | 0.08977 |
| svm | 0.85141 | rf | 0.09367 |

**Table 9: Computed performances using assay 3for the super learner with equal weights (slew), the super learner module (sl), and the set of machines used for the reduced features.**

| Reduced machine | Reduced machine AUC | Reduced machine | Reduced machine BS |
|---|---|---|---|
| slew | 0.9236 | slew | 0.07809 |
| gam | 0.92318 | gam | 0.07839 |
| sl | 0.92278 | sl | 0.07844 |
| glmboost | 0.92197 | glmboost | 0.0785 |
| en | 0.92159 | en | 0.07855 |
| lr | 0.92158 | lr | 0.07859 |
| mars | 0.92024 | mars | 0.07911 |
| gbm | 0.9199 | gbm | 0.07912 |
| rf | 0.91583 | svm | 0.08761 |
| svm | 0.87544 | rf | 0.09051 |

**Table 10: Computed performances using assay 4 for the super learner with equal weights (slew), the super learner module (sl), and the set of machines used for the reduced features.**

| Reduced machine | Reduced machine AUC | Reduced machine | Reduced machine BS |
|---|---|---|---|
| gam | 0.9271 | sl | 0.07823 |
| sl | 0.92678 | gam | 0.0784 |
| slew | 0.92614 | slew | 0.07876 |
| gbm | 0.92494 | en | 0.07926 |
| glmboost | 0.92328 | glmboost | 0.07932 |
| lr | 0.92307 | lr | 0.0794 |
| en | 0.92294 | gbm | 0.08053 |
| rf | 0.91849 | mars | 0.08352 |
| mars | 0.9127 | svm | 0.08444 |
| svm | 0.87492 | rf | 0.0905 |

When reviewing the results estimated for troponin assay 1 as illustrated in table 7, the super learner with equal weights (slew) achieved the highest AUC score with 0.91673 and the super learner (sl) achieved the second highest AUC score with 0.91638. Furthermore, the slew achieved the lowest Brier score with 0.07947, meaning that the slew may have the lowest error rate for assay 1.

When reviewing the results estimated for troponin assay 2 as illustrated in table 8, slew achieved the second highest AUC score with 0.90986 and the sl achieved the highest AUC score with 0.90987. Furthermore, the slew achieved the lowest Brier score with 0.08386, meaning. When reviewing the results estimated for troponin assay 3 as illustrated in table 9, the slew achieved the highest AUC score with 0.9236 and the sl achieved the third highest AUC score with 0.92278. Furthermore, the slew achieved the lowest Brier score with 0.07809, meaning that the slew may have the lowest error rate for troponin assay 3.

When reviewing the results estimated for troponin assay 4 as illustrated in table 10, the gam achieved the highest AUC score with 0.9271, sl achieved the second highest AUC score with 0.92678 and the slew achieved the third highest AUC score with 0.92614. Furthermore, the sl achieved the lowest Brier score with 0.07823, meaning that the sl has the lowest error rate for assay 4. Hence, since the slew achieved the highest AUC scores for troponin assay 1 and troponin assay 2, and had the lowest Brier scores for troponin assay 1, troponin assay 2 and troponin assay 3, the slew may be considered the best performing machine.

The performance of the slew may be further compared with an external database. The reduced feature set associated with assay 1 of the BACC may be used. The external database may be the stenoCardia database. The stenoCardia database may also include datasets. A feature set comprising an equal amount of datasets as the reduced feature and associated with assay 1 may be extracted from the stenoCardia. The reduced feature set and the extracted set may each be divided into 10 partitions. 9 of the 10 partitions may be used to fit the slew, while a remaining partition not used for fitting the machine may be used to estimate the performance. For the reduced feature set of the BACC, the slew may compute a AUC score of 0.9167 and a Brier score of 0.0795. For the extracted feature set of the stenoCardia, the slew may compute a AUC score of 0.9601 and a Brier score of 0.0644.

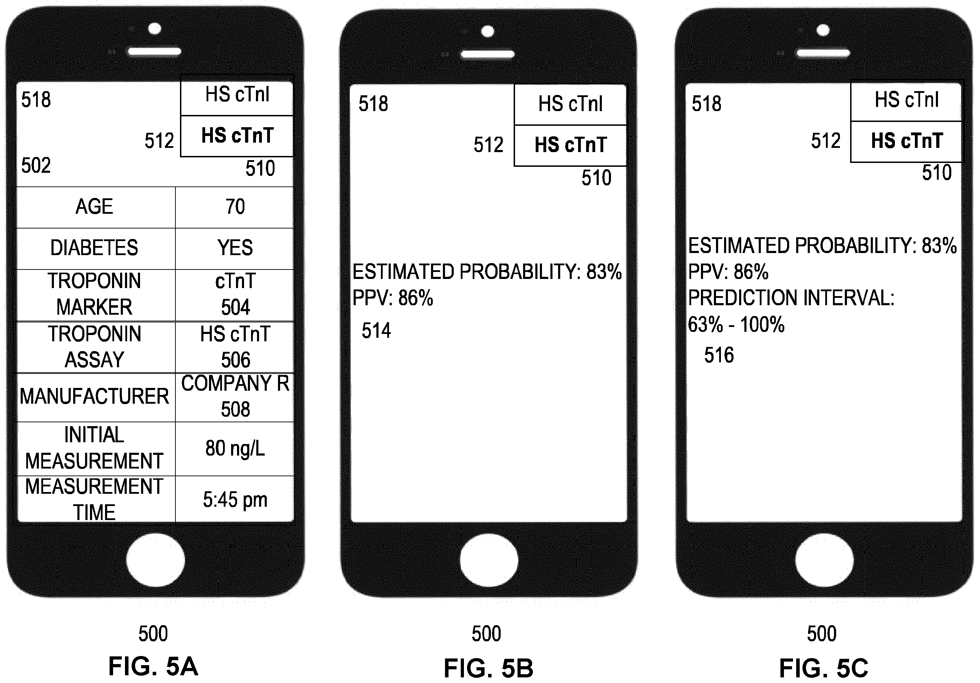

FIGs. 5A to 5C illustrate a schematic overview of preselecting a troponin assay applicable in embodiments of the present disclosure. FIGs. 5A to 5C show a computing device 500, which may correspond to the computing device 100 of Fig. 1.

The computing device 500 receives a set of vital parameters 502, which may include troponin data of a subject measured with an initial troponin assay. A preselector component of the computing device 500 may scan the received troponin data. By scanning the troponin data of the received set of vital parameters 502, the preselector component may estimate a measured troponin marker 504, a name of the troponin assay 506 and a manufacturer of the troponin assay 508. For example, the troponin marker cTnT 504 may have been measured using the troponin assay HS cTnT 506, which may have been provided by the manufacturer COMPANY R 508. Each troponin assay from the set of troponin assays 510 may include information including a name of the troponin assay, the manufacturer that may provide the troponin assay, and which troponin marker the troponin assay may measure. In response to scanning the received troponin data, the preselector component may estimate that the troponin assay HS cTnT 512 from the set of troponin assays 510 is able to measure the troponin marker cTnT and that HS cTnT is also provided by the manufacturer COMPANY 508. Therefore, the preselector component may preselect the troponin assay by sending a request to the selector of the computing device, which may in response select the preselected troponin assay.

FIG. 5B illustrates the preselecting of a troponin assay further causing the computing device to estimate a probability of myocardial infarction. When the selector selects the troponin assay from the set of troponin assays, in response to the preselector preselecting the most likely used troponin assay, a plurality of estimation modules forming a super learner module may be provided for estimating the probability of myocardial infarction for the subject. This may be performed, for example, as illustrated in figure 2. Hence, the preselector component may cause the computing device to estimate the probability without any further user interaction. For the received set of vital parameters 502, the computing device may estimate a probability of 86% of myocardial infarction. Furthermore, if the estimated probability was ruled-in by cutoffs associated with the troponin assay, a positive predictive value may be 86%. The estimated probability and the PPV may be displayed on the user interface 518 of the computing device 500, as shown in item 514.

According to another embodiment as illustrated in FIG. 5C, the computing device may have been configured to automatically estimate a prediction interval for the estimated probability. The estimated prediction interval of between 63% and 100% may be displayed in item 516 on the user interface 518 of the computing device 500.

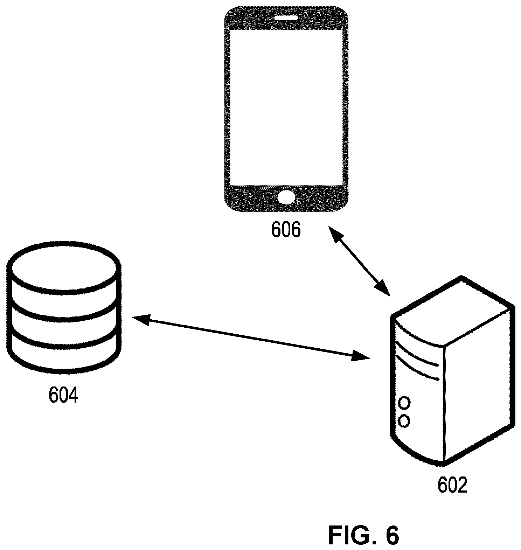

FIG. 6 shows a schematic view of a system according to an embodiment of the present disclosure. The system may comprise a server 602, a database 604 and a computing device 606. The computing device 606 may correspond to any one of the computing devices 100 or 500 as shown in FIGs. 1, and 5A to 5C. The server 602 may be configured to estimate estimation modules that may constitute a super learner module, according to one or more embodiments of the present disclosure, such as discussed, for example, with respect to FIG. 3.

When estimating machines for the one or more super learner modules, the server may use data of the database 604. The database may be the BACC database or any other suitable databased related to troponin measurements. The computing device 606 may be connected to the server 602 via a wireless connection. When the computing device 606 connects to the server 602 for the first time, the server 602 may provide several estimation modules to a repository of the computing device 606. The provided one or more plurality of estimation modules may be updated and/or verified at each subsequent connection. Furthermore, the server may train one or more of the plurality of estimations modules with a set of equal weights.

The server 602 may receive a request to create a plurality of estimation modules from an extension component of the computing device 606. When the server 606 receives the request, the server 606 may retrieve machines that the 1MM and/or the 2MM comprise and train them with data of the database associated with a troponin marker included in the request. The new trained plurality of estimation modules may be provided to the repository of the computing device 606. The plurality of estimation modules may be stored in the repository of the computing device.

The server 606 may further estimate residuals and store the estimated residuals in one or more pre-computed tables. When the computing device 602 connects to the server, the one or more pre-computed tables may be provided to the computing device 602 so that the computing device may use the pre-computed tables to estimate a prediction interval. The provided plurality of pre-computed tables may be updated and/or verified at each subsequent connection.

Referring back to FIG. 3, according to another embodiment relating to items 306 and 308, the machines can be estimated in an updated version of the BACC database consisting of 2719 subjects before exclusions and of 2575 subjects after exclusions. An additional troponin assay (assay 5) was included in the analyses. In place of the generalized additive model machines (gam) a logistic regression modeling continuous variables with restricted cubic splines (lrrcs) was used. A log loss (LogLoss) was used as performance measure. Rankings for the performance estimates of the machines based on the reduced features for the LogLoss scores have been computed, similar to the results shown in Tables 5 and 6.

In one example, the assays may include Architect STAT High Sensitive Troponin-I (Abbott Diagnostics) as assay 1; Elecsys Troponin T hs STAT (Roche) as assay 2; Atellica IM TnIH (Siemens) as assay 3; PATHFAST hs-cTnI (Mitsubishi) as assay 4; and Access hsTnI (Beckman Coulter) as assay 5. However, it is to be understood that other existing and emerging central-laboratory or point-of-care troponin assays, like for example the Siemens VTLi POC assay, and other combinations of assays can be used and the present disclosure is not limited to a particular selection or combination of assays.

**Table 11: Ranking of the 1MM machines using the reduced features based on the computed LogLoss scores.**

| Machine | Assay 1 ranking | Assay 2 ranking | Assay 3 ranking | Assay 4 ranking | Assay 5 ranking | Summed ranks |
|---|---|---|---|---|---|---|
| lrrcs | 2 | 1 | 1 | 1 | 3 | 8 |
| gbm | 4 | 2 | 2 | 3 | 1 | 12 |
| glmboost | 1 | 3 | 5 | 5 | 4 | 18 |
| en | 3 | 4 | 3 | 4 | 5 | 19 |
| mars | 5 | 6 | 7 | 2 | 2 | 22 |
| lr | 6 | 5 | 4 | 6 | 6 | 27 |
| svm | 7 | 7 | 6 | 7 | 7 | 34 |
| rf | 8 | 8 | 8 | 8 | 8 | 40 |

The abbreviations utilized in the Tables represent the following machines: logistic regression machine with restricted cubic splines (lrrcs); gradient boosting machine (gbm); generalized linear machine with boosting (glmboost); elastic net machine (en); multivariate adaptive regression splines machine (mars); logistic regression machine (lr); support vector machine (svm); and random forest machine (rf).

**Table 12: Ranking of the 2MM machines using the reduced features based on the computed LogLoss scores.**

| Machine | Assay 1 ranking | Assay 2 ranking | Assay 3 ranking | Assay 4 ranking | Assay 5 ranking | Summed ranks |
|---|---|---|---|---|---|---|
| gbm | 1 | 1 | 1 | 1 | 1 | 5 |
| lrrcs | 2 | 3 | 2 | 2 | 3 | 12 |
| mars | 3 | 2 | 3 | 4 | 2 | 14 |
| rf | 4 | 4 | 4 | 3 | 4 | 19 |
| svm | 5 | 5 | 5 | 7 | 7 | 29 |
| glmboost | 7 | 6 | 7 | 5 | 5 | 30 |
| en | 6 | 7 | 6 | 6 | 6 | 31 |
| lr | 8 | 8 | 8 | 8 | 8 | 40 |

The ranking in tables 11 and 12 is applied similar to the ranking in tables 5 and 6. The best performance is ranked with a 1 and the subsequent best performances are ranked with 2, 3, 4, etc. The assigned ranks of each machine for each assay may be summed up and the machine with the lowest rank is considered as the best performing machine.

Based on the LogLoss scores as shown in table 11, the best-performing machines were chosen for 1MM, including lrrcs, gbm, glmboost, en, and mars. The third and fourth ranking machines in Table 11, namely the generalized linear machine with boosting (glmboost) and the elastic net machine (en) revealed high correlation of estimated probabilities. Therefore, only the best of these two, i.e. glmboost, was used. The next-ranking multivariate adaptive regression splines machine (mars) was included as replacement.

In a preferred embodiment, the machines of the 1MM of the super learner module may comprise a logistic regression machine with restricted cubic splines, a gradient boosting machine, a generalized linear machine with boosting, and a multivariate adaptive regression splines machine.

Based on the LogLoss scores as shown in table 12, the best-performing machines were chosen for 2MM, including gbm, lrrcs, mars, and rf. In a preferred embodiment, the machines of the 2MM of the super learner module may comprise a gradient boosting machine, a logistic regression machine with restricted cubic splines, a multivariate adaptive regression splines machine, and a random forest machine.

Super learners with equal weights using the 2, 3 and the 4 best ranked machines were computed for 1MM and 2MM. For 1MM the super learner with equal weight using 4 variables (slew4) showed the best performance based on the LogLoss compared to the super learner using 2 (slew2) and 3 (slew3) machines. For 2MM, slew3 performed always better than slew4 and slew2 with the exception of assay 2, where slew2 performed better than both slew 2 and slew4. For this reason, slew4 was used for 1MM and slew3 for 2MM. Performance of the 1MM and 2MM machines using the reduced set of features are shown in the subsequent tables.

**Table 13: Computed performances (LogLoss) using assays 1 to 5 for the 1MM super learners with equal weights (slew4, slew3 and slew2), and the set of machines used for the reduced features.**

| Reduced feature machines assay 1 | Reduced features LogLoss scores assay 1 | Reduced feature machines assay 2 | Reduced features LogLoss scores assay 2 | Reduced feature machines assay 3 | Reduced features LogLoss scores assay 3 |
|---|---|---|---|---|---|
| slew4 | 0.26293 | slew4 | 0.26346 | slew4 | 0.25282 |
| slew3 | 0.26473 | slew2 | 0.26464 | slew2 | 0.25438 |
| slew2 | 0.26561 | slew3 | 0.26471 | slew3 | 0.25443 |
| glmboost | 0.26862 | lrrcs | 0.26727 | lrrcs | 0.25545 |
| lrrcs | 0.26877 | gbm | 0.26932 | gbm | 0.25834 |
| en | 0.26944 | glmboost | 0.27123 | en | 0.26131 |
| gbm | 0.27160 | en | 0.27161 | lr | 0.26155 |
| mars | 0.27402 | lr | 0.27192 | glmboost | 0.26158 |
| lr | 0.27416 | mars | 0.27264 | svm | 0.27041 |
| svm | 0.27878 | svm | 0.27904 | mars | 0.27436 |
| rf | 0.28764 | rf | 0.28699 | rf | 0.28030 |

**Table 13 (continued): Computed performances (LogLoss) using assays 1 to 5 for the 1MM super learners with equal weights (slew4, slew3 and slew2), and the set of machines used for the reduced features.**

| Reduced feature machines assay 4 | Reduced features LogLoss scores assay 4 | Reduced feature machines assay 5 | Reduced features LogLoss scores assay 5 |
|---|---|---|---|
| slew4 | 0.24506 | slew4 | 0.26324 |
| lrrcs | 0.24679 | slew2 | 0.26442 |
| slew3 | 0.24769 | slew3 | 0.26537 |
| slew2 | 0.24788 | gbm | 0.26750 |
| mars | 0.25313 | mars | 0.26829 |
| gbm | 0.25340 | lrrcs | 0.26990 |
| en | 0.25445 | glmboost | 0.27437 |
| glmboost | 0.25484 | en | 0.27441 |
| lr | 0.25502 | lr | 0.27457 |
| svm | 0.26842 | svm | 0.28282 |
| rf | 0.27080 | rf | 0.28837 |

Table 13 illustrates that the super learner based on four machines (slew4) shows the best performance for 1MM. The four machines consist of the best ranking in Table 11, wherein en was replaced by mars. Table 13 also shows that the performance of slew4 is even better than the performance of single machines.

**Table 14: Computed performances (LogLoss) using assays 1 to 5 for the 2MM super learners with equal weights (slew4, slew3 and slew2), and the set of machines used for the reduced features.**

| Reduced feature machines assay 1 | Reduced features LogLoss scores assay 1 | Reduced feature machines assay 2 | Reduced features LogLoss scores assay 2 | Reduced feature machines assay 3 | Reduced features LogLoss scores assay 3 |
|---|---|---|---|---|---|
| slew3 | 0.20190 | slew3 | 0.16677 | gbm | 0.18732 |
| slew4 | 0.20235 | slew4 | 0.17014 | slew2 | 0.18769 |
| slew2 | 0.20272 | slew2 | 0.17063 | slew3 | 0.18831 |
| gbm | 0.20285 | gbm | 0.17246 | slew4 | 0.19066 |
| lrrcs | 0.22192 | mars | 0.17559 | lrrcs | 0.20083 |
| mars | 0.22275 | lrrcs | 0.19705 | mars | 0.20350 |
| rf | 0.22360 | rf | 0.20278 | rf | 0.21444 |
| svm | 0.23708 | svm | 0.20541 | svm | 0.22982 |
| en | 0.24460 | glmboost | 0.23237 | en | 0.23593 |
| glmboost | 0.24478 | en | 0.23250 | glmboost | 0.23622 |
| lr | 0.25133 | lr | 0.23713 | lr | 0.23637 |

**Table 14 (continued): Computed performances (LogLoss) using assays 1 to 5 for the 2MM super learners with equal weights (slew4, slew3 and slew2), and the set of machines used for the reduced features.**

| Reduced feature machines assay 4 | Reduced features LogLoss scores assay 4 | Reduced feature machines assay 5 | Reduced features LogLoss scores assay 5 |
|---|---|---|---|
| slew3 | 0.20488 | slew3 | 0.21747 |
| slew4 | 0.20625 | slew4 | 0.21922 |
| slew2 | 0.20653 | slew2 | 0.22208 |
| gbm | 0.20677 | gbm | 0.22465 |
| lrrcs | 0.22370 | mars | 0.22852 |
| rf | 0.22678 | lrrcs | 0.23237 |
| mars | 0.22871 | rf | 0.23916 |
| glmboost | 0.23140 | glmboost | 0.24755 |
| en | 0.23417 | en | 0.24851 |
| svm | 0.23841 | svm | 0.25131 |
| lr | 0.24214 | lr | 0.25275 |

Table 14 illustrates that the super learner based on three machines (slew3) shows the best performance for 2MM, with the single exception of one assay (assay 3). There, the gradient boosting machine (gbm) and the super learner based on two machines (slew2) performs slightly better. However, across all assays, the performance of slew3 is the best for 2MM.

In summary, tables 13 and 14 show that the super learner based on four machines (slew4) and the super learner based on three machines (slew3) for 1MM and 2MM, respectively perform better that any single machine. The data shows that super learner modules can be used to improve estimation of the probability of myocardial infarction of subjects.

The features disclosed in the above description and figures may be relevant to the realization of the invention as defined by the following claims.

## Claims

1. A computing device (100) for estimating the probability of myocardial infarction, the computing device comprising:
a receiver (102) configured to receive a set of vital parameters of a subject, the set of vital parameters including troponin data of the subject, the troponin data of the subject being measured using an initial troponin assay;
a selector (104) configured to select a troponin assay from a set of troponin assays, wherein the selector selects the troponin assay in response to a manual, pre-set or an automated selection of the troponin assay, wherein the selection corresponds to the initial troponin assay;
a repository (106) configured to provide a combination of a plurality of estimation modules corresponding to the selected troponin assay, wherein the combination of the plurality of estimation modules constitute a super learner module, wherein the repository stores a plurality of combinations of a plurality of estimation modules, wherein each stored combination of the plurality of estimation modules is associated with a troponin assay from the set of troponin assays, wherein the repository is configured to provide the combination of the plurality of estimation modules associated with the selected troponin assay, when the selector selects the troponin assay from the set of troponin assays, wherein a combination of the plurality of estimation modules constituting a super learner module corresponds to selected best performing or ranked estimation modules; and
at least one processor (108) configured to use the provided combination of the plurality of estimation modules to estimate the probability of myocardial infarction of the subject using the set of vital parameters including the troponin data of the subject.

2. The computing device according to claim 1, wherein the troponin data includes at least one troponin measurement of a troponin marker, the troponin measurement including a measurement value of the troponin marker and a timestamp of the troponin measurement.

3. The computing device according to claim 1 or 2, wherein the at least one processor is further configured to estimate a prediction interval for the probability of myocardial infarction.

4. The computing device according to claim 3, wherein the prediction interval is estimated by:
setting a coverage level;
drawing equal-sized folds of datasets in at least a part of a database for cross-validation;
forming cross-validated observations with prediction variability for each equal-sized fold;
determining a mean and standard deviation of the cross-validated observations; and
estimating the prediction interval using the coverage level, and the mean and standard deviation of the created observations.

5. The computing device according to claim 4, wherein forming cross-validated observation comprises, for each equal-sized fold:
training each of the plurality of estimation modules using the equal-sized folds without the respective equal-sized fold;
making predictions for all data in the respective equal-sized fold;
estimating residuals for all data in the respective equal-sized fold using the predictions; and
randomly drawing a number of data without replacement from the respective equal-sized fold, to form the cross-validated observations and a prediction error, using the fold-specific predicted observation and the respective residual of the randomly drawn data.

6. The computing device according to claim 4 or 5, wherein residuals are stored in one or more pre-computed tables in the repository.

7. The computing device according to any one of the preceding claims, wherein the super learner module corresponds to a 1 measurement model if the troponin data includes one troponin measurement, and wherein the super learner module corresponds to a 2 measurement model or to a 1 measurement model if the troponin data includes at least two troponin measurements.

8. The computing device according to claim 7, wherein the 1 measurement model comprises estimation modules of differing types including one or more of a gradient boosting machine, a generalized additive machine and a generalized boosted regression machine, and wherein the 2 measurement model comprises estimation modules of differing types including one or more of a generalized additive machine, a generalized boosted regression machine and a random forest machine.

9. The computing device according to claim 7 or 8, wherein one or more super learner modules are fitted by a server, using a set of equal weights.

10. The computing device according to any one of the preceding claims, further comprising an extension component (110) configured to add a further troponin assay to the set of troponin assays, wherein a combination of a plurality of further estimation modules corresponding to the further troponin assay is stored in the repository.

11. The computing device according to any one of the preceding claims, further comprising a preselector component (112) configured to preselect a troponin assay corresponding to the initial troponin assay based on at least the troponin data of the subject.

12. The computing device according to any one of the preceding claims, wherein the computing device further comprises a user interface (114), wherein the user interface is configured to receive input of a user and provide at least the estimated probability to the user.

13. The computing device according to any one of the preceding claims, wherein the computing device is a portable computing device, including one of a mobile communication device, a smart device, a smart watch, or a personal digital assistant.

14. A system, comprising:
a server; and
at least one computing device for estimating the probability of myocardial infarction according to any one of the preceding claims.

15. The system according to claim 14, further comprising:
a network, wherein the at least one computing device is connected to the server via the network, wherein the server is configured to provide the combination of the plurality of estimation modules constituting the super learner module to the repository of the at least one computing device.

16. The system according to claim 14 or 15, further comprising a database configured to store datasets related to vital parameters including troponin data.

17. The system according to any one of the claims 14 to 16, wherein the server is further configured to store estimated residuals in one or more pre-computed tables for one or more machines and/or super learner modules.

## Patentansprüche

1. Eine Computervorrichtung (100) zur Abschätzung der Wahrscheinlichkeit eines Myokardinfarkts, wobei die Computervorrichtung umfasst:
einen Empfänger (102), der konfiguriert ist, einen Satz von Vitalparametern einer Person zu empfangen, wobei der Satz von Vitalparametern Troponindaten der Person umfasst, wobei die Troponindaten der Person unter Verwendung eines initialen Troponin-Assays gemessen werden;
einen Selektor (104), der konfiguriert ist, einen Troponin-Assay aus einem Satz von Troponin-Assays auszuwählen, wobei der Selektor den Troponin-Assay in Antwort auf eine manuelle, voreingestellte oder automatisierte Auswahl des Troponin-Assays auswählt, wobei die Auswahl dem initialen Troponin-Assay entspricht;
einen Speicher (106), der konfiguriert ist, eine Kombination einer Mehrzahl von Schätzmodulen bereitzustellen, die dem ausgewählten Troponin-Assay entsprechen, wobei die Kombination der Mehrzahl von Schätzmodulen ein Super-Learner-Modul bildet, wobei der Speicher eine Mehrzahl von Kombinationen einer Mehrzahl von Schätzmodulen speichert, wobei jede gespeicherte Kombination der Mehrzahl von Schätzmodulen einem Troponin-Assay aus dem Satz von Troponin-Assays zugeordnet ist, wobei der Speicher konfiguriert ist, die dem ausgewählten Troponin-Assay zugeordnete Kombination der Mehrzahl von Schätzmodulen bereitzustellen, wenn der Selektor den Troponin-Assay aus dem Satz von Troponin-Assays auswählt, wobei eine Kombination der Mehrzahl von Schätzmodulen, die ein Super-Learner-Modul bilden, den ausgewählten Schätzmodulen mit der besten Leistung oder dem höchsten Rang entspricht; und
mindestens einen Prozessor (108), der konfiguriert ist, die bereitgestellte Kombination der Mehrzahl von Schätzmodulen zu verwenden, um die Wahrscheinlichkeit eines Myokardinfarkts bei der Person unter Verwendung des Satzes von Vitalparametern, einschließlich der Troponindaten der Person, zu schätzen.

2. Die Computervorrichtung gemäß Anspruch 1, wobei die Troponindaten mindestens eine Troponinmessung eines Troponinmarkers umfassen, wobei die Troponinmessung einen Messwert des Troponinmarkers und einen Zeitstempel der Troponinmessung umfasst.

3. Die Computervorrichtung gemäß Anspruch 1 oder 2, wobei der mindestens eine Prozessor ferner konfiguriert ist, ein Vorhersageintervall für die Wahrscheinlichkeit eines Myokardinfarkts zu schätzen.

4. Die Computervorrichtung gemäß Anspruch 3, wobei das Vorhersageintervall geschätzt wird durch:
Festlegen eines Abdeckungsgrades;
Entnehmen gleich großer Teilmengen von Datensätzen von mindestens einem Teil einer Datenbank zur Kreuzvalidierung;
Bilden kreuzvalidierter Beobachtungen mit Vorhersagevariabilität für jede gleich große Teilmenge;
Ermitteln eines Mittelwerts und einer Standardabweichung der kreuzvalidierten Beobachtungen; und
Schätzen des Vorhersageintervalls unter Verwendung des Abdeckungsgrades sowie des Mittelwerts und der Standardabweichung der erstellten Beobachtungen.

5. Die Computervorrichtung gemäß Anspruch 4, wobei das Bilden kreuzvalidierter Beobachtungen für jede gleich große Teilmenge umfasst:
Trainieren jedes der Mehrzahl von Schätzmodulen unter Verwendung der gleich großen Teilmengen ohne die jeweilige gleich große Teilmenge;
Erstellen von Vorhersagen für alle Daten in der jeweiligen gleich großen Teilmenge;
Schätzen von Residuen für alle Daten in der jeweiligen gleich großen Teilmenge unter Verwendung der Vorhersagen; und
zufälliges Entnehmen einer Anzahl von Daten ohne Zurücklegen aus der jeweiligen gleich großen Teilmenge, um die kreuzvalidierten Beobachtungen und einen Vorhersagefehler zu bilden, unter Verwendung der teilmengenspezifischen vorhergesagten Beobachtung und des jeweiligen Residuums der zufällig entnommenen Daten.

6. Die Computervorrichtung gemäß Anspruch 4 oder 5, wobei Residuen in einer oder mehreren vorberechneten Tabellen im Speicher gespeichert sind.

7. Die Computervorrichtung gemäß einem der vorangehenden Ansprüche, wobei das Super-Learner-Modul einem 1-Messungs-Modell entspricht, wenn die Troponindaten eine Troponinmessung umfassen, und wobei das Super-Learner-Modul einem 2-Messungs-Modell oder einem 1-Messungs-Modell entspricht, wenn die Troponindaten mindestens zwei Troponinmessungen umfassen.

8. Die Computervorrichtung gemäß Anspruch 7, wobei das 1-Messungs-Modell Schätzmodule unterschiedlicher Typen umfasst, einschließlich einer oder mehrerer einer Gradient-Boosting-Maschine, einer generalisierten additiven Maschine und einer generalisierten Boosted-Regressionsmaschine, und wobei das 2-Messungs-Modell Schätzmodule unterschiedlicher Typen umfasst, einschließlich einer oder mehrerer einer generalisierten additiven Maschine, einer generalisierten Boosted-Regressionsmaschine und einer Random-Forest-Maschine.

9. Die Computervorrichtung gemäß Anspruch 7 oder 8, wobei ein oder mehrere Super-Learner-Module von einem Server unter Verwendung eines Satzes gleicher Gewichte angepasst werden.

10. Die Computervorrichtung gemäß einem der vorangehenden Ansprüche, ferner umfassend eine Erweiterungskomponente (110), die konfiguriert ist, einen weiteren Troponin-Assay zum Satz von Troponin-Assays hinzuzufügen, wobei eine Kombination aus einer Mehrzahl weiterer Schätzmodule, die dem weiteren Troponin-Assay entsprechen, im Speicher gespeichert ist.

11. Die Computervorrichtung gemäß einem der vorangehenden Ansprüche, die ferner eine Vorauswahl-Komponente (112) umfasst, die konfiguriert ist, einen dem initialen Troponin-Assay entsprechenden Troponin-Assay basierend auf mindestens den Troponindaten der Person vorzuselektieren.

12. Die Computervorrichtung gemäß einem der vorangehenden Ansprüche, wobei die Computervorrichtung ferner eine Benutzerschnittstelle (114) umfasst, wobei die Benutzerschnittstelle konfiguriert ist, Eingaben eines Benutzers zu empfangen und dem Benutzer mindestens die geschätzte Wahrscheinlichkeit bereitzustellen.

13. Die Computervorrichtung gemäß einem der vorangehenden Ansprüche, wobei die Computervorrichtung eine tragbare Computervorrichtung ist, die ein mobiles Kommunikationsgerät, ein Smart-Gerät, eine Smartwatch oder einen persönlichen digitalen Assistenten umfasst.

14. Ein System, umfassend:
einen Server; und
mindestens eine Computervorrichtung zur Abschätzung der Wahrscheinlichkeit eines Myokardinfarkts gemäß einem der vorangehenden Ansprüche.

15. Das System gemäß Anspruch 14, ferner umfassend:
ein Netzwerk, wobei die mindestens eine Computervorrichtung über das Netzwerk mit dem Server verbunden ist, wobei der Server konfiguriert ist, die Kombination der Mehrzahl von Schätzmodulen, die das Super-Learner-Modul bilden, dem Speicher der mindestens einen Computervorrichtung bereitzustellen.

16. Das System gemäß Anspruch 14 oder 15, ferner umfassend eine Datenbank, die konfiguriert ist, Datensätze in Bezug auf Vitalparameter einschließlich Troponindaten zu speichern.

17. Das System gemäß einem der Ansprüche 14 bis 16, wobei der Server ferner konfiguriert ist, geschätzte Residuen in einer oder mehreren vorberechneten Tabellen für eine oder mehrere Maschinen und/oder Super-Learner-Module zu speichern.

## Revendications

1. Dispositif informatique (100) d'estimation de la probabilité d'infarctus du myocarde, le dispositif informatique comprenant :
un récepteur (102) configuré pour recevoir un ensemble de paramètres vitaux d'un sujet, l'ensemble de paramètres vitaux incluant des données de troponine du sujet, les données de troponine du sujet étant mesurées à l'aide d'un dosage initial de troponine ;
un sélecteur (104) configuré pour sélectionner un dosage de troponine parmi un ensemble de dosages de troponine, dans lequel le sélecteur sélectionne le dosage de troponine en réponse à une sélection manuelle, prédéfinie ou automatisée du dosage de troponine, dans lequel la sélection correspond au dosage de troponine initial ;
un référentiel (106) configuré pour fournir une combinaison d'une pluralité de modules d'estimation correspondant au dosage de troponine sélectionné, dans lequel la combinaison de la pluralité de modules d'estimation constitue un module super apprenant, dans lequel le référentiel stocke une pluralité de combinaisons d'une pluralité de modules d'estimation, dans lequel chaque combinaison stockée de la pluralité de modules d'estimation est associée à un dosage de troponine de l'ensemble de dosages de troponine, dans lequel le référentiel est configuré pour fournir la combinaison de la pluralité de modules d'estimation associés au dosage de troponine sélectionné, lorsque le sélecteur sélectionne le dosage de troponine dans l'ensemble de dosages de troponine, dans lequel une combinaison de la pluralité de modules d'estimation constituant un module super apprenant correspond aux modules d'estimation les plus performants ou les mieux classés sélectionnés ; et
au moins un processeur (108) configuré pour utiliser la combinaison fournie de la pluralité de modules d'estimation pour estimer la probabilité d'infarctus du myocarde du sujet en utilisant l'ensemble de paramètres vitaux incluant les données de troponine du sujet.

2. Dispositif informatique selon la revendication 1, dans lequel les données de troponine incluent au moins une mesure de troponine d'un marqueur de troponine, la mesure de troponine incluant une valeur de mesure du marqueur de troponine et un horodatage de la mesure de troponine.

3. Dispositif informatique selon la revendication 1 ou 2, dans lequel l'au moins un processeur est en outre configuré pour estimer un intervalle de prédiction pour la probabilité d'infarctus du myocarde.

4. Dispositif informatique selon la revendication 3, dans lequel l'intervalle de prédiction est estimé par :
le réglage d'un niveau de couverture ;
le tirage de folds de taille égale d'ensembles de données dans au moins une partie d'une base de données pour validation croisée ;
la formation d'observations à validation croisée avec une variabilité de prédiction pour chaque fold de taille égale ;
la détermination d'une moyenne et d'un écart-type des observations à validation croisée ; et
l'estimation de l'intervalle de prédiction à l'aide du niveau de couverture, et de la moyenne et de l'écart-type des observations créées.

5. Dispositif informatique selon la revendication 4, dans lequel la formation d'une observation validée croisée comprend, pour chaque fold de taille égale :
l'entraînement de chacun de la pluralité de modules d'estimation à l'aide des folds de taille égale sans le fold de taille égale respectif ;
la réalisation de prédictions pour toutes les données dans le fold de taille égale respectif ;
l'estimation de résidus pour toutes les données dans le fold de taille égale respectif à l'aide des prédictions ; et
le tirage aléatoire d'un certain nombre de données sans remplacement à partir du fold de taille égale respectif, pour former les observations à validation croisée et une erreur de prédiction, à l'aide de l'observation prédite spécifique au fold et du résidu respectif des données tirées aléatoirement.

6. Dispositif informatique selon la revendication 4 ou 5, dans lequel des résidus sont stockés dans une ou plusieurs tables précalculées dans le référentiel.

7. Dispositif informatique selon l'une quelconque des revendications précédentes, dans lequel le module super apprenant correspond à un modèle de mesure 1 si les données de troponine incluent une mesure de troponine, et dans lequel le module super apprenant correspond à un modèle de mesure 2 ou à un modèle de mesure 1 si les données de troponine incluent au moins deux mesures de troponine.

8. Dispositif informatique selon la revendication 7, dans lequel le modèle de mesure 1 comprend des modules d'estimation de types différents incluant une ou plusieurs parmi une machine d'augmentation de gradient, une machine additive généralisée et une machine de régression augmentée généralisée, et dans lequel le modèle de mesure 2 comprend des modules d'estimation de types différents incluant une ou plusieurs parmi une machine additive généralisée, une machine de régression augmentée généralisée et une machine de forêt aléatoire.

9. Dispositif informatique selon la revendication 7 ou 8, dans lequel un ou plusieurs modules super apprenants sont aménagés dans un serveur, à l'aide d'un ensemble de poids égaux.

10. Dispositif informatique selon l'une quelconque des revendications précédentes, comprenant en outre un composant d'extension (110) configuré pour ajouter un dosage de troponine supplémentaire à l'ensemble de dosages de troponine, dans lequel une combinaison d'une pluralité de modules d'estimation supplémentaires correspondant au dosage de troponine supplémentaire est stockée dans le référentiel.

11. Dispositif informatique selon l'une quelconque des revendications précédentes, comprenant en outre un composant de présélection (112) configuré pour présélectionner un dosage de troponine correspondant au dosage de troponine initial sur la base d'au moins les données de troponine du sujet.

12. Dispositif informatique selon l'une quelconque des revendications précédentes, dans lequel le dispositif informatique comprend en outre une interface utilisateur (114), dans lequel l'interface utilisateur est configurée pour recevoir une entrée d'un utilisateur et fournir au moins la probabilité estimée à l'utilisateur.

13. Dispositif informatique selon l'une quelconque des revendications précédentes, dans lequel le dispositif informatique est un dispositif informatique portable, incluant l'un parmi un dispositif de communication mobile, un dispositif intelligent, une montre connectée ou un assistant numérique personnel.

14. Système, comprenant :
un serveur ; et
au moins un dispositif informatique pour estimer la probabilité d'infarctus du myocarde selon l'une quelconque des revendications précédentes.

15. Système selon la revendication 14, comprenant en outre :
un réseau, dans lequel l'au moins un dispositif informatique est connecté au serveur via le réseau, dans lequel le serveur est configuré pour fournir la combinaison de la pluralité de modules d'estimation constituant le module super apprenant au référentiel de l'au moins un dispositif informatique.

16. Système selon la revendication 14 ou 15, comprenant en outre une base de données configurée pour stocker des ensembles de données relatifs à des paramètres vitaux incluant des données de troponine.

17. Système selon l'une quelconque des revendications 14 à 16, dans lequel le serveur est en outre configuré pour stocker des résidus estimés dans une ou plusieurs tables précalculées pour une ou plusieurs machines et/ou un ou plusieurs modules super apprenants.
